# FASCICULE DE BREVET EUROPEEN

(11) **EP 4 225 295 B1**
(45) Date de publication et mention de la délivrance du brevet: **13.11.2024**
(21) Numéro de dépôt: 21798748.6
(22) Date de dépôt: 30.09.2021
(51) Int. Cl.: A61K 31/198, A61K 38/02, A61P 29/02, C07K 5/10

(54) **NOUVEAUX PEPTOIDES ET LEUR UTILISATION DANS LA PREVENTION OU LE TRAITEMENT DE LA DOULEUR CHRONIQUE**
NEUE PEPTOIDE UND IHRE VERWENDUNG ZUR VORBEUGUNG ODER BEHANDLUNG CHRONISCHER SCHMERZEN
NOVEL PEPTOIDS AND USE THEREOF FOR PREVENTING OR TREATING CHRONIC PAIN

(30) Priorité: 06.10.2020 FR 2010186
(43) Date de publication de la demande: 16.08.2023
(73) Titulaire: Université Clermont Auvergne, 63000 Clermont-Ferrand (FR); Centre National de la Recherche Scientifique, 75016 Paris (FR); Sigma Clermont, 63170 Aubière (FR); Institut National de la Santé et de la Recherche Médicale (INSERM), 75013 Paris (FR); Université de Montpellier, 34090 Montpellier (FR)
(72) Inventeur: BUSSEROLLES, Jérôme, 03800 SAULZET (FR); BOURINET, Emmanuel, 34270 SAINT-MATHIEU-DE-TREVIERS (FR); TAILLEFUMIER, Claude, 63320 CHIDRAC (FR); ROY, Olivier, 63800 COURNON D'AUVERGNE (FR); NAUTON, Lionel, 63320 CHAMPEIX (FR); AISSOUNI, Youssef, 63200 RIOM (FR)
(74) Mandataire: Novagraaf Technologies
(86) Numéro de dépôt international: PCT/FR2021/051694
(87) Numéro de publication internationale: WO 2022/074313

(56) Documents cités:
- EP-A1- 2 289 886
- HRUBY VICTOR J ED - MERCER JULIAN: "Multivalent peptide and peptidomimetic ligands for the treatment of pain without toxicities and addiction", PEPTIDES, vol. 116, 2019, pages 63 - 67, XP085693658, ISSN: 0196-9781, DOI: 10.1016/J.PEPTIDES.2019.02.004

## Description

### Domaine technique de l'invention

L'invention concerne de nouvelles molécules peptidomimétiques (ou peptoïdes, également appelés hybrides peptide-peptoïde ou peptomères) pour la prévention et/ou le traitement de la douleur chronique, notamment celle résultant de neuropathies périphériques (e.g. induites par chimiothérapie).

Dans la description ci-dessous, les références entre crochets ([ ]) renvoient à la liste des références à la fin du texte.

### Etat de l'art

La douleur chronique touche 1,5 milliard de personnes dans le monde pour lesquelles la réponse thérapeutique est clairement insuffisante (Finnerup NB et al., 2015) [1]. Des études épidémiologiques récentes ont estimé que la douleur chronique touche environ 20 % des adultes et 50 % des personnes âgées (von Hehn et al., 2012) [2]. Au niveau mondial, 60 % des personnes souffrant de douleurs sont moins aptes à travailler et 20 % déclarent avoir perdu leur emploi à cause de la douleur. Parallèlement, 20 milliards d'unités de médicaments analgésiques ont été vendues en 2010, contre 14 milliards en 2005 (+9,3 %). En 2017, le marché mondial de la douleur a atteint 61,05 milliards de dollars et devrait rapporter 77,13 milliards de dollars d'ici 2023, enregistrant un taux de croissance annuel composé de 4% de 2017 à 2023.

Les douleurs neuropathiques concernent environ 7% de la population générale et 25% des patients atteints de douleurs chroniques. Elles sont de deux types : la douleur neuropathique périphérique (selon son origine, elle peut concerner les territoires d'un plexus, d'une racine, d'un tronc ou être plus diffuse dans le cadre de polyneuropathies) et la douleur neuropathique centrale (lésions affectant les voies sensitives ou du contrôle de la douleur). Les neuropathies périphériques induites par la chimiothérapie, effets indésirables fréquents de nombreux agents anticancéreux, se caractérisent par des douleurs chroniques importantes affectant durablement la qualité de vie des patients et entrainant des adaptations posologiques avec le risque d'une efficacité clinique amoindrie. La pharmacopée antalgique, qui s'appauvrit (retrait du Di-antalvic en 2011), se caractérise par des traitements anciens et souvent mal tolérés.

L'innovation est en panne depuis plus de 50 ans, la majorité des nouveaux médicaments proposés étant de simples reformulations ou associations de médicaments existants. De récentes méta-analyses montrent qu'aucun traitement contre la douleur neuropathique n'est réellement efficace à ce jour à l'exception de la gabapentine et de la duloxétine avec un score d'efficacité encore faible (Hershman et al., 2014) [3].

La famille des canaux ioniques HCN (Ludwig et al., Nature 1998 ; Santoro et al., 1998 ; Seifert et al., 1999) [4-6], qui comprend quatre membres HCN1-4, pourrait offrir d'excellentes possibilités de développement de nouveaux médicaments destinés à réduire la fréquence cardiaque et de nouveaux analgésiques. Les canaux HCN sont largement répartis dans les voies de la douleur et jouent un rôle important dans le développement et le maintien de la douleur neuropathique (Dunlop et al., 2009 ; Lewis et al., 2011) [7, 8]. Il a été démontré que les bloqueurs HCN non sélectifs atténuent les symptômes de la douleur dans des modèles de douleur neuropathique chez les rongeurs (Descoeur et al., 2011 ; Young et al., 2014) [9, 10]. Leurs effets secondaires cardiaques et visuels (e.g. bloqueurs pan-HCN) limitent toutefois la traduction clinique de leur utilisation pour traiter la douleur neuropathique.

Il est intéressant de noter que la fonction des canaux HCN est étroitement régulée par une sous-unité auxiliaire, la protéine d'interaction Rab8b (TRIP8b) qui n'est pas exprimée dans le coeur. Cette protéine cytoplasmique se lie à l'extrémité C-terminale des sous-unités du canal HCN via deux sites de contact (Lewis et al., 2009) [11]. Par ailleurs, la structure atomique de la région tétratricopeptide (TPR) de TRIP8b en complexe avec un peptide de 6 acides aminés représentant l'extrémité C-terminale de HCN2 a été déterminée par cristallographie aux rayons X (Bankston et al., 2012 )[12].

Dans le contexte actuel, la majorité des travaux s'intéressant à développer des inhibiteurs agissant directement sur les canaux HCN, le ciblage de TRIP8b et/ou de l'interaction TRIP8b-HCN pourrait offrir une nouvelle possibilité pour moduler l'activité du HCN dans les états de douleur sans risque d'affecter les fonctions cardiaques et visuelles.

Des peptoïdes et peptidomimétiques sont également envisagés pour la prévention et/ou le traitement de la douleur chronique (Hruby, Peptides, 116 : 63-67, 2019 ; Demande de Brevet EP 2289886) [29, 30].

### Description de l'invention

La présente invention repose sur l'hypothèse que la perturbation des interactions TRIP8b-HCN conduisant à une diminution des courants (Ih) induits par les canaux HCN, pourrait avoir un effet antalgique dans des modèles de neuropathies périphériques aiguë et chroniques induites par l'oxaliplatine chez le rongeur reproduisant fidèlement la symptomatologie humaine.

La preuve de l'efficacité de cette stratégie a dans un premier temps été validée à l'aide de NUCC-5953, une molécule issue d'un criblage d'une banque de 20 000 molécules, capable de perturber l'interaction TRIP8b-HCN (Han et al., 2015) [13]. Les Inventeurs ont ainsi démontré que l'hypersensibilité aiguë au froid induite par l'oxaliplatine est réversée de manière dose-dépendante par l'injection intrathécale (Figure 2) ou systémique (Figure 3) de NUCC-5953. Aucune altération de l'activité locomotrice n'a été observée. Les données préliminaires ont également montré que l'administration de NUCC-5953 réduit l'adressage à la membrane, et donc l'activité de HCN1 et 2 observée chez les animaux neuropathiques (Figure 5B). De plus, l'application *in vitro* de NUCC-5953 n'a montré aucun effet sur les courants induits par l'activation des HCN dans les cellules cardiaques (contrairement à des bloqueurs non spécifiques des canaux HCN, Figure 6).

Les inventeurs ont alors décidé de concevoir des bloqueurs spécifiques de l'interaction TRIP8b-HCN en se basant sur le mode d'interaction des deux protéines. Toutefois si les interactions protéine-protéine (PPI) jouent un rôle essentiel à tous les niveaux du fonctionnement cellulaire, ce qui en font des cibles thérapeutiques incontestables, la conception de puissants inhibiteurs de PPI demeure un réel défi. Les molécules de faible poids moléculaire bien que largement criblées, sont peu adaptées pour rivaliser avec des interactions impliquant de larges surfaces (>800Å). En outre pour concevoir des inhibiteurs efficaces, il est important de pouvoir mimer le segment de reconnaissance protéine/protéine « hot segment » dans sa conformation bioactive. Les peptides sont les composés de choix pour concevoir ces nouveaux inhibiteurs à condition de pouvoir résoudre les problèmes de stabilités métabolique et conformationnelle, mais également de pénétration cellulaire pour atteindre des cibles intracellulaires.

Les inventeurs se sont alors tournés vers une chimie peptidomimétique via la synthèse de peptoïdes (oligomères de glycine N-substitués) qui sont particulièrement adaptés au développement d'inhibiteurs ciblant une interaction protéine-protéine, et qui peuvent également être dénommés peptomères (*i.e*. des oligomères combinant des monomères « peptoides » et des monomères « amino-acides » - Ostergaard et al 1997 [17] - soit des hybrides peptide-peptoide).

Ce sont en effet des mimes peptidiques synthétiques dont la nature oligomérique permet une grande modularité en termes de taille et de diversité chimique.

Ils permettent un design structural fin et possèdent une meilleure biodisponibilité que les peptides (résistance aux protéases, meilleure perméabilité membranaire, squelette non-immunogénique).

Les Inventeurs ont donc conçu de nouveaux peptoïdes ciblant l'interaction entre les domaines TPR de TRIP8b et l'extrémité C-terminale de HCN, capables d'exercer un effet analgésique dans un modèle de neuropathie aiguë induite par l'oxaliplatine sans effets cardiaques ou visuels indésirables.

La présente invention a donc pour objet un peptoïde de formule générale (I) suivante : 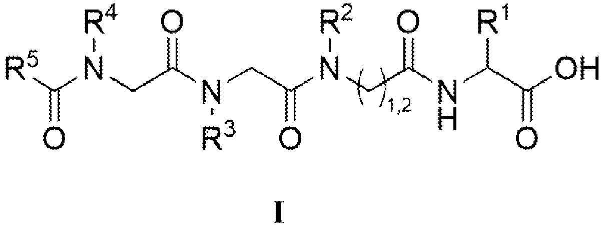 où
**R¹** est de manière indépendante CH₃ (L-Ala), CH(CH₃)₂ (L-Val), CH(CH₃)CH₂CH₃ (L-Ile), CH₂CH(CH₃)₂ (L-Leu and D-Leu), CH₂C(CH₃)₃, C(CH₃)₃, CH₂(cyclobutyl) ;
**R²**, **R³**, et **R⁴** sont les chaînes latérales des unités peptoïdes incorporées séquentiellement au cours de la synthèse à partir d'amines primaires ;
**R²** et **R³** sont choisis de manière indépendante parmi : CH₂CH(CH₃)₂, CH₂CH₂NH₂, CH₂CH₂OH, CH₂CH₂CH₂OH, CH₂CH(OH)CH₃, CH₂CONH₂, CH₂CH₂CONH₂. Les chaînes latérales présentant une fonction alcool ou amine ont été incorporées aux oligomères sous forme protégée : éther de silyle pour la fonction alcool et tert-butyl carbamate (Boc) pour la fonction amine ;
**R⁴** est un groupe aliphatique ou aromatique choisi parmi : 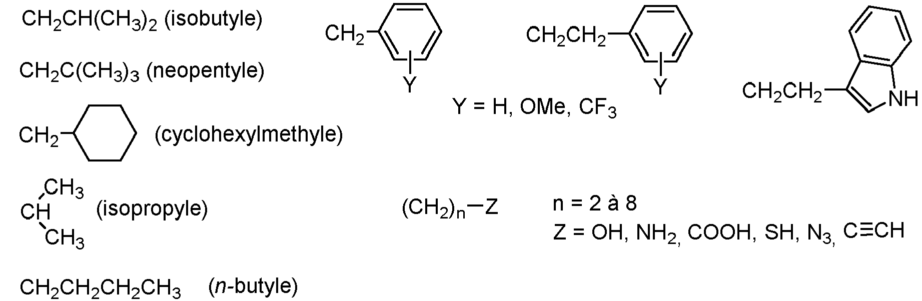
**R⁵** est de manière indépendante CH₃, CH(CH₃)₂, CH₂C₆H₅, C₆H₄X (où X en position ortho, méta ou para est de manière indépendante H, OMe, CF₃, CH₃, CH₂CH₃, F, Br, Cl, NO₂), 3-indolyl, 3-quinolinyl.

Avantageusement, **R⁴** est un groupe aliphatique ou aromatique choisi parmi :

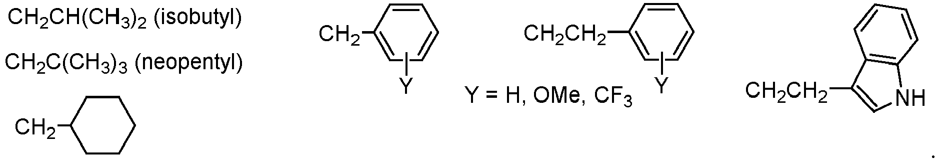

Avantageusement, **R⁵** est de manière indépendante CH₃, CH₂C₆H₅, C₆H₄X (où X en position ortho, méta ou para est de manière indépendante H, OMe, CF₃, CH₃, CH₂CH₃, F, Br, Cl), 3-indolyl, 3-quinolinyl.

Avantageusement, les peptoïdes de la présente invention sont choisis parmi les peptoïdes de formule générale (I) dans laquelle :
- **R¹** est de manière indépendante CH₃ (L-Ala), CH(CH₃)₂ (L-Val), CH(CH₃)CH₂CH₃ (L-Ile), CH₂CH(CH₃)₂ (L-Leu and D-Leu), CH₂C(CH₃)₃, C(CH₃)₃, CH₂(cyclobutyl) ;
- **R²**, **R³**, et **R⁴** sont les chaînes latérales des unités peptoïdes incorporées séquentiellement au cours de la synthèse à partir d'amines primaires ;
- **R²** et **R³** sont choisis de manière indépendante parmi : CH₂CH₂NH₂, CH₂CH₂OH, CH₂CH₂CH₂OH, CH₂CH(OH)CH₃, CH₂CONH₂, CH₂CH₂CONH₂. Les chaînes latérales présentant une fonction alcool ou amine ont été incorporées aux oligomères sous forme protégée : éther de silyle pour la fonction alcool et tert-butyl carbamate (Boc) pour la fonction amine ;
- **R⁴** est un groupe aliphatique ou aromatique choisi parmi : 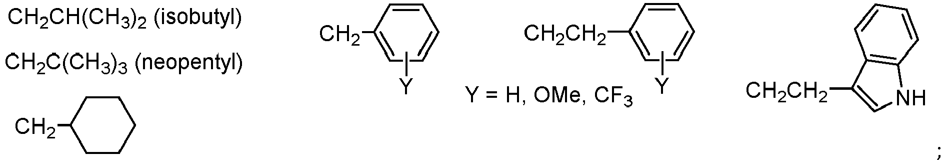
- **R⁵** est de manière indépendante CH₃, CH₂C₆H₅, C₆H₄X (où X en position ortho, méta ou para est de manière indépendante H, OMe, CF₃, CH₃, CH₂CH₃, F, Br, Cl), 3-indolyl, 3-quinolinyl.

De préférence, les peptoïdes de la présente invention, de formule générale (I), comportent 2 atomes de carbone sur la seconde unité (en partant de l'extrémité C-terminale).

Avantageusement, les peptoïdes de la présente invention sont choisis parmi les peptoides de formule générale (I') suivante : 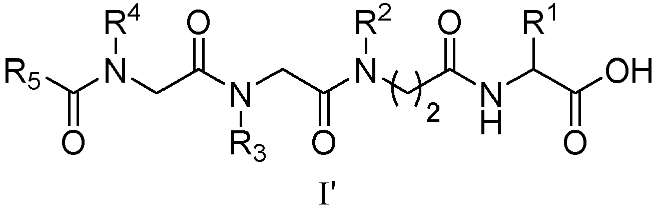 où
**R¹**, **R², R³, R⁴** et **R⁵** sont tels que définis ci-dessus.

Selon un mode de réalisation particulier, les peptoïdes de la présente invention ont les formules suivantes :

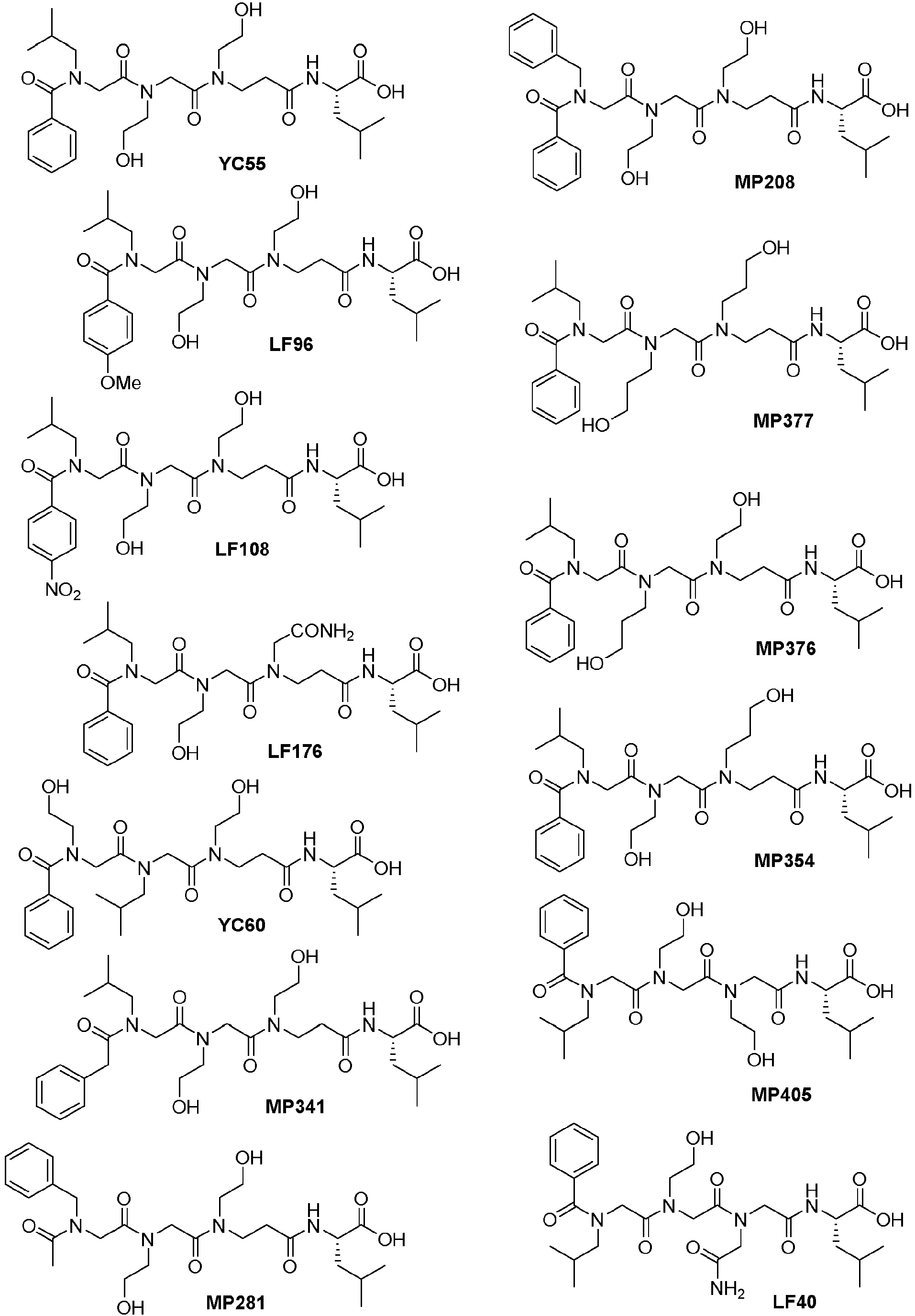

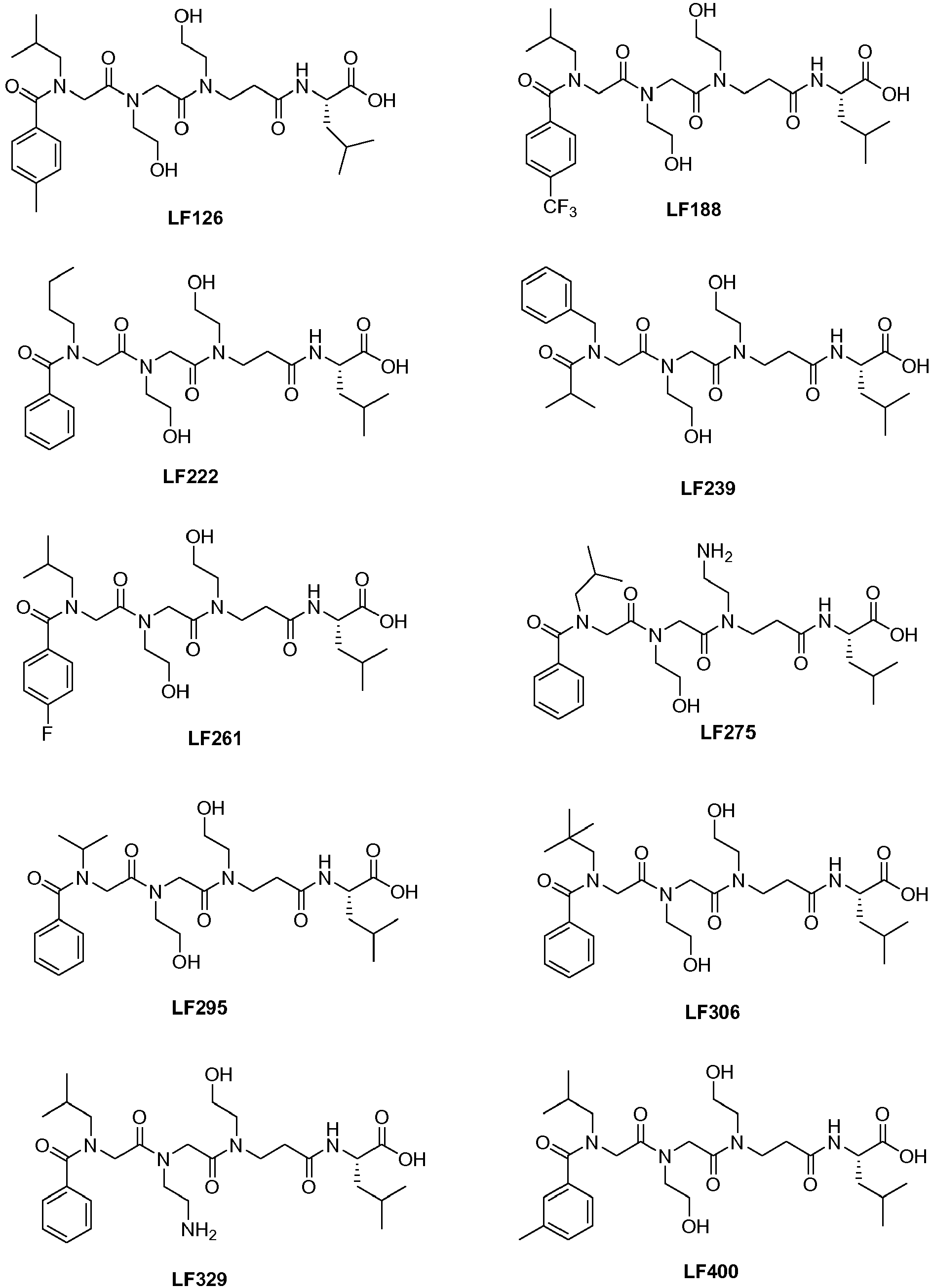

La présente invention a également pour objet un intermédiaire de synthèse du peptoïde de formule générale (I), le dit peptoïde comprenant 3 unités. Ledit intermédiaire de synthèse est un peptoïde de formule générale (II) suivante : 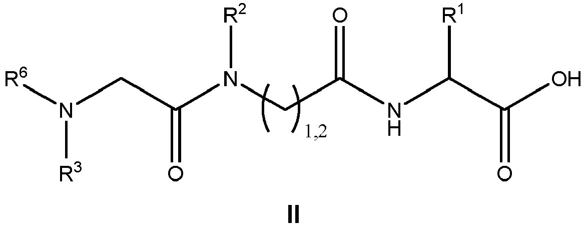 où
**R¹**, **R²** et **R³** sont tels que définis ci-dessus ;
**R⁶** est H, **R³**, CO**R⁵** (où **R⁵** est tel que défini ci-dessus) ou **R⁷**;
**R⁷** est de manière indépendante un groupe parmi :
   (CH₂)_{w}CH₃ alkyle (w = 1-4)

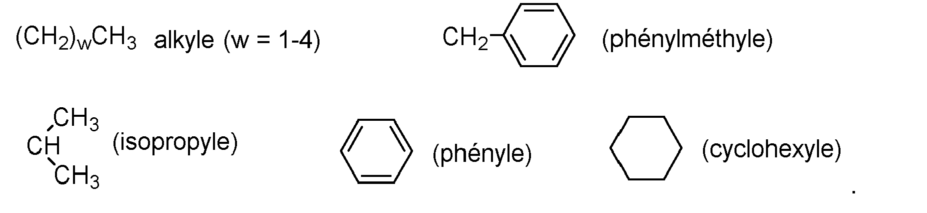

En particulier, la présente invention a pour objet les peptoïdes de formule (II) dans laquelle **R⁶** est H.

En particulier, la présente invention a pour objet les peptoïdes de formule (II) dans laquelle **R⁶** est CO**R⁵**.

En particulier, la présente invention a pour objet les peptoïdes de formule (II) dans laquelle **R⁶** est **R⁷**.

En particulier, la présente invention a pour objet les peptoïdes de formule (II) dans laquelle **R⁶** est **R³**.

Avantageusement, les peptoïdes selon l'invention peuvent être choisis parmi les peptoïdes de formules générales (IIa), (IIb), (IIc) et (IId) suivantes : 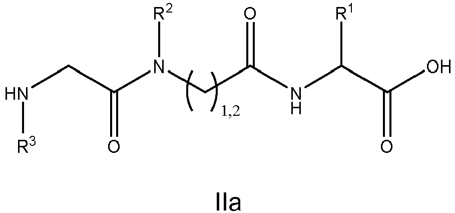 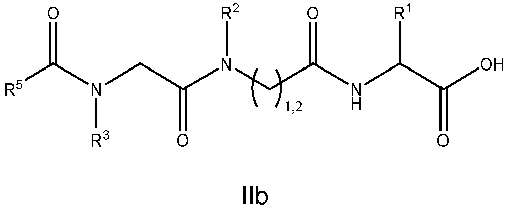 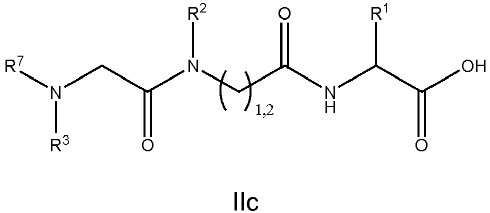 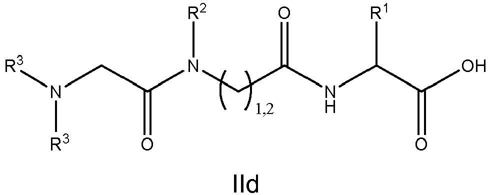 où
**R¹**, **R²**, **R³**, **R⁵** et **R⁷** sont tels que définis ci-dessus.

De préférence, les peptoïdes de la présente invention, de formule (II), (IIa), (IIb), (IIc) ou (IId), comportent 2 atomes de carbone sur la seconde unité (en partant de l'extrémité C-terminale). Avantageusement, les peptoïdes sont choisis parmi les peptoides de formules (II'), (II'a), (II'b), (II'c) ou (II'd) suivantes : 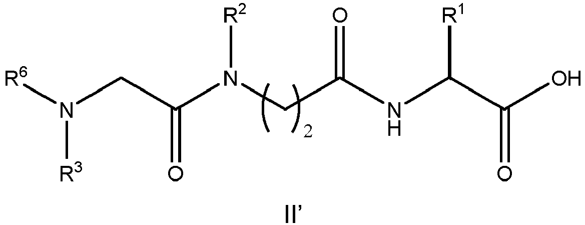 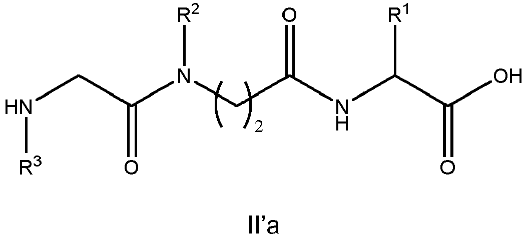 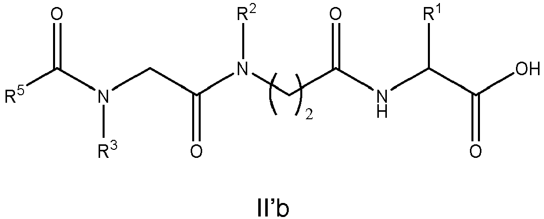 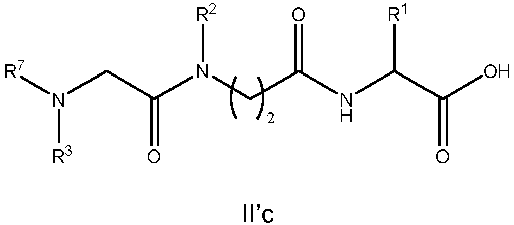 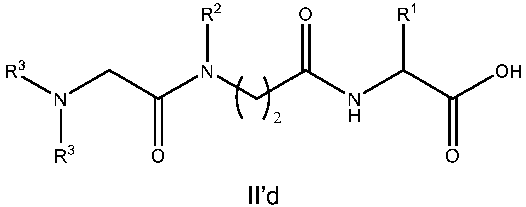 où
**R¹, R², R³**, **R⁵**, **R⁶** et **R⁷** sont tels que définis ci-dessus.

La présente invention a également pour objet une composition pharmaceutique comprenant un peptoïde selon la présente invention, et un véhicule pharmaceutiquement acceptable.

La présente invention a également pour objet un peptoïde ou une composition pharmaceutique selon la présente invention pour une utilisation comme médicament.

La présente invention a également pour objet un peptoïde ou une composition pharmaceutique selon l'invention pour une utilisation dans la prévention ou le traitement de la douleur chronique, en particulier de la douleur chronique d'origine neuropathique, plus particulièrement de la douleur neuropathique périphérique par exemple induite par chimiothérapie.

Dans un contexte où l'innovation thérapeutique dans le domaine des antalgiques est en panne, les peptoïdes de la présente invention et leur potentiel thérapeutique peuvent avoir un impact important sur l'amélioration de la qualité de vie des patients souffrant de douleurs neuropathiques induites par des anticancéreux et éventuellement de leur survie. De plus, les canaux HCN étant également impliqués dans l'étiologie de diverses douleurs d'origine neuropathique et inflammatoire, les peptoïdes de la présente invention ouvrent la voie à de nouvelles classes de médicaments antalgiques actifs plus largement sur différents types de douleur chronique.

### Brève description des figures

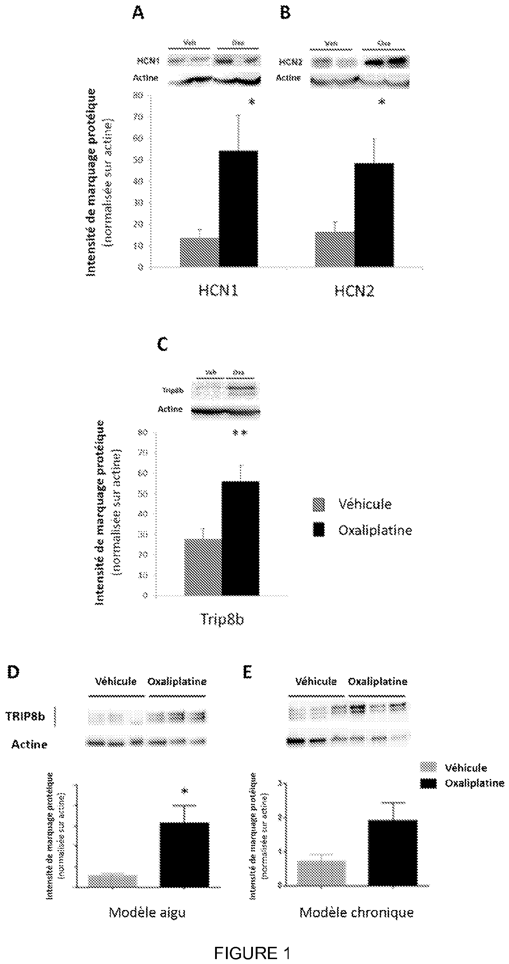

La figure 1 représente l'expression des protéines HCN1 (A), HCN2 (B) et TRIP8b (C) dans les neurones DRG 4 jours après l'administration de véhicule ou d'oxaliplatine (6mg/kg, i.p.). L'expression de la protéine TRIP8b dans la corne dorsale de la moelle épinière 4 jours après l'administration de véhicule ou d'oxaliplatine (6mg/kg, i.p.) (D) ou après l'administration répétée (deux fois par semaine pendant trois semaines) de véhicule ou d'oxaliplatine (E) N=3/groupe, test de Mann-Whitney.

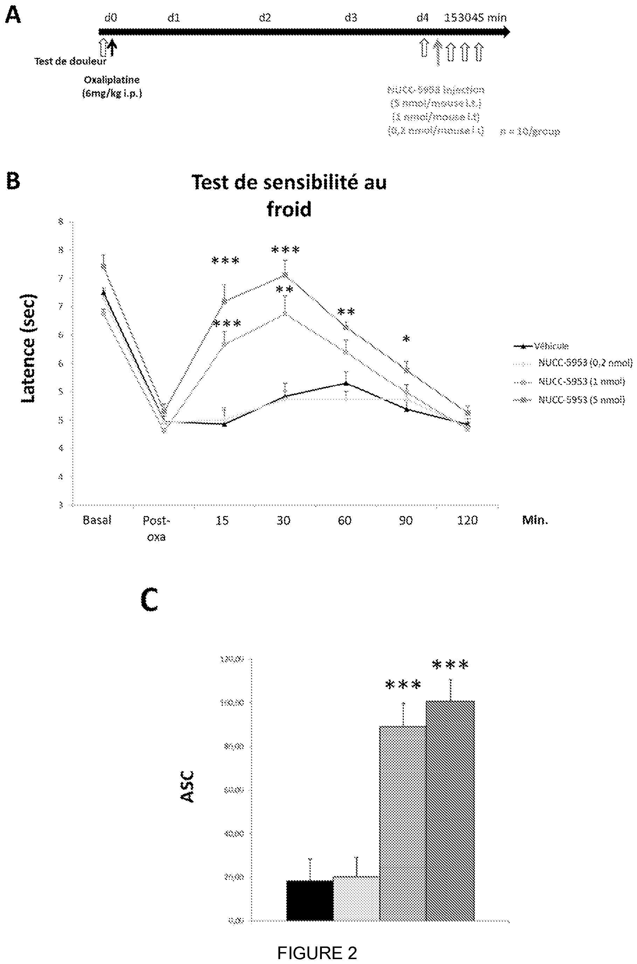

La figure 2 représente (A) conception de l'étude (B) évolution temporelle de la moyenne +/- SEM du seuil de latence à l'immersion de la patte (en sec) et (C) aire sous la courbe (ASC, sec.min) de l'effet analgésique dépendant de la dose chez des souris OIPN (souris modèle de neuropathie induite par oxaliplatine) (n=8) traitées avec le véhicule ou NUCC5953 (0.2, 1 ou 5 µg, i.t.) ; * p<0.05, ** p<0.01, *** p<0.001, vs le groupe veh., ANOVA à deux voies et test *post hoc* de Tukey.

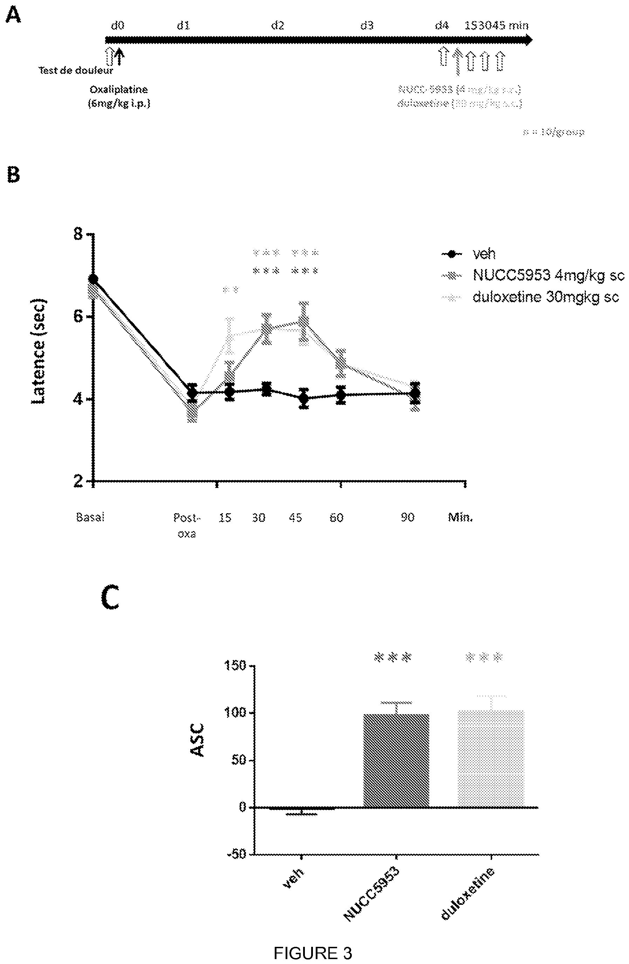

La figure 3 représente (A) conception de l'étude (B) évolution temporelle de la moyenne +/- SEM du seuil de latence à l'immersion de la patte (en sec) et (C) aire sous la courbe (ASC, sec.min) de l'effet analgésique dépendant de la dose chez des souris OIPN (n=8) traitées avec le véhicule, NUCC5953 (4mg/kg, s.c.) ou duloxetine (30mg/kg, s.c.) ; * p<0.05, ** p<0.01, *** p<0.001, vs le groupe veh., ANOVA à deux voies et test *post hoc* de Tukey.

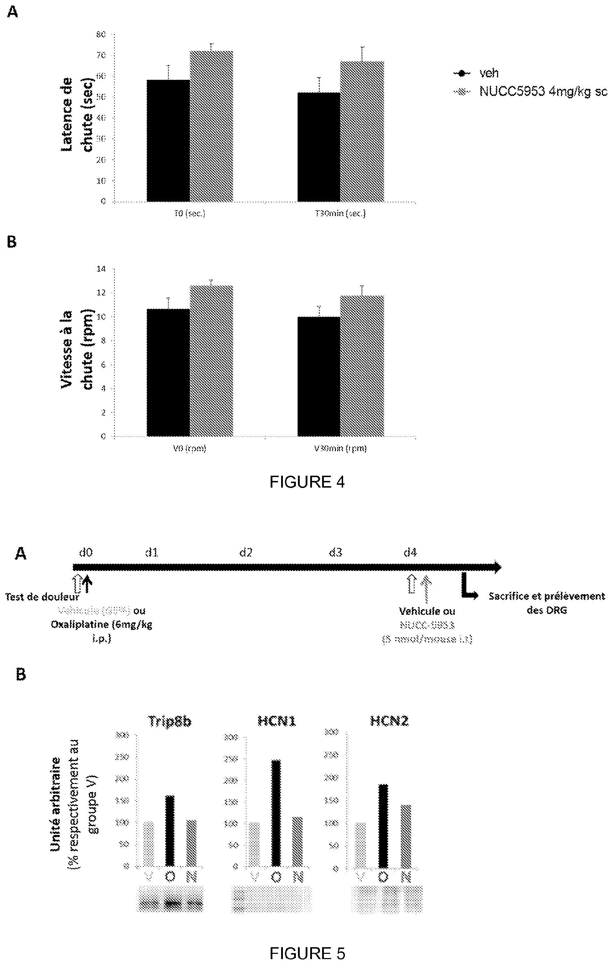

La figure 4 représente les histogrammes montrant la latence à la chute (A) et à la vitesse de chute (B) de souris traitées avec le véhicule ou NUCC5953 (4 mg/kg, s.c.) soumise à un test de rotarod à vitesse croissante (de 4 à 40 tours/minute pendant 10 min).
La figure 5 représente (A) conception de l'étude (B) l'expression (membranaire) subcellulaire de HCN1, HCN2 et TRIP8b dans les DRGs d'animaux traités avec le véhicule (V), l'oxaliplatine (O) ou l'oxaliplatine+NUCC5953 (5 nmol, i.t.) (N).

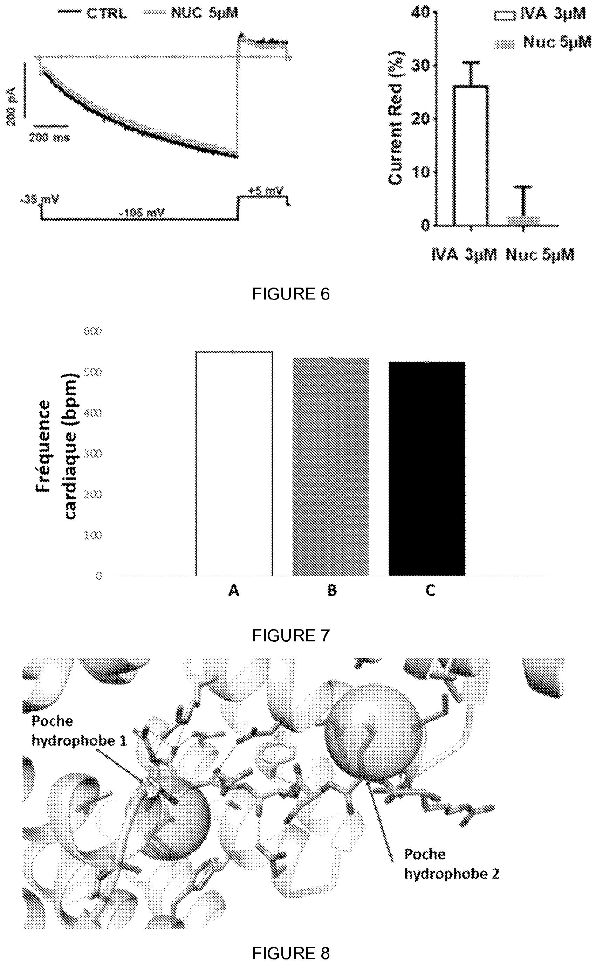

La figure 6 représente l'absence d'effet de NUCC5953 sur l'amplitude du courant If contrairement à l'ivabradine (IVA).
La figure 7 représente le rythme cardiaque des souris (RC) : (A) valeur de référence, (B) moyenne de la fréquence cardiaque pendant 30 minutes après l'injection de NUCC5953 (4 mg/kg, s.c.) et (C) valeur moyenne à la fin des 30 minutes ; les valeurs sont une moyenne +/- SEM (n=3).
La figure 8 représente la dynamique moléculaire de TRIP8b en interaction avec le peptide SNL.

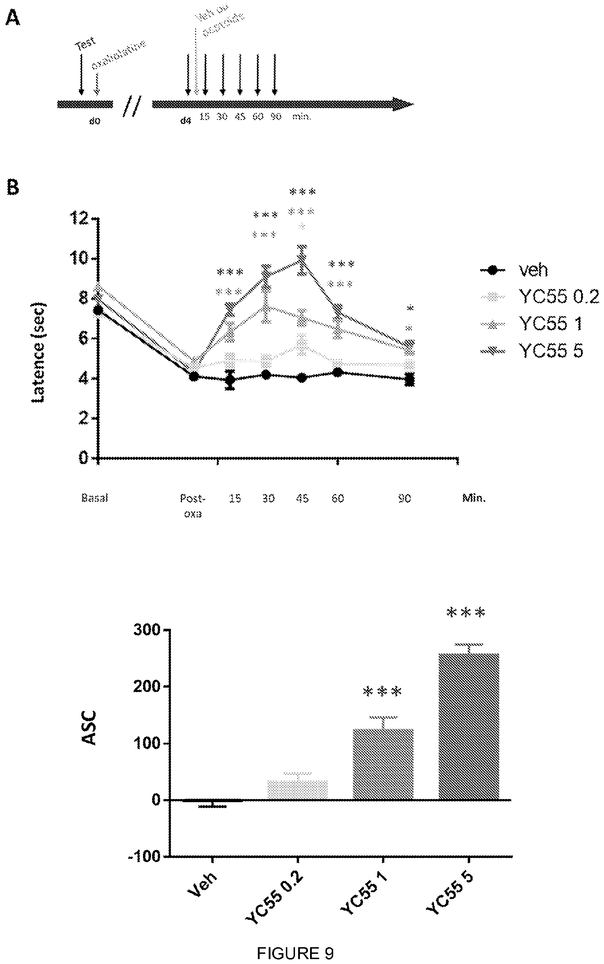

La figure 9 représente (A) conception de l'étude (B) évolution temporelle de la moyenne +/- SEM du seuil de latence à l'immersion de la patte (en sec) et (C) aire sous la courbe (ASC, sec.min) de l'effet analgésique dépendant de la dose chez des souris OIPN (n=8) traitées avec le véhicule ou le peptoïde YC55 (0.2, 1 ou 5 µg, i.t.) ; * p<0.05, ** p<0.01, *** p<0.001, vs le groupe veh., ANOVA à deux voies et test *post hoc* de Tukey.

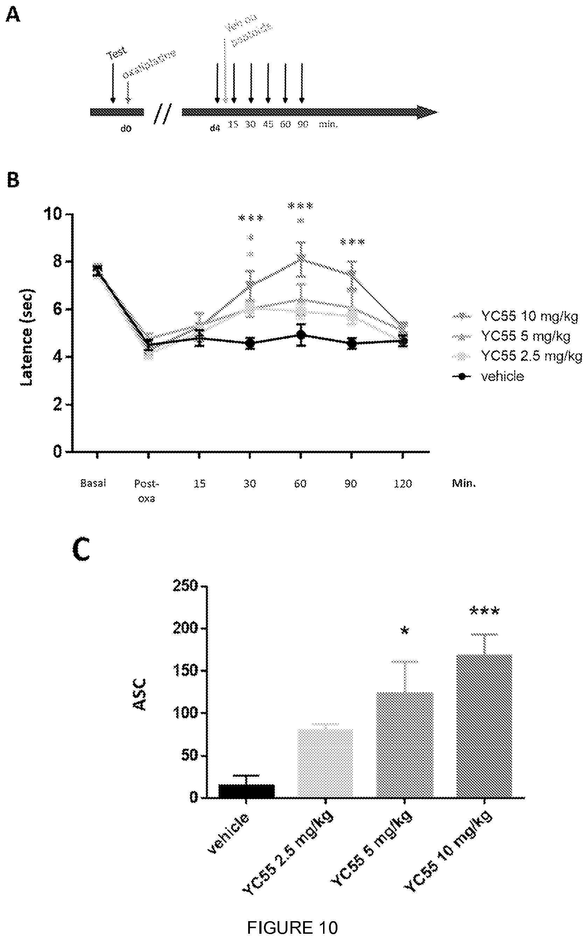

La figure 10 représente (A) conception de l'étude (B) évolution temporelle de la moyenne +/- SEM du seuil de latence à l'immersion de la patte (en sec) et (C) aire sous la courbe (ASC, sec.min) de l'effet analgésique dépendant de la dose chez des souris OIPN (n=8) traitées avec le véhicule ou le peptoïde YC55 (2.5, 5 ou 10 mg/kg, s.c.) ; * p<0.05, ** p<0.01, *** p<0.001, vs le groupe veh., ANOVA à deux voies et test *post hoc* de Tukey.

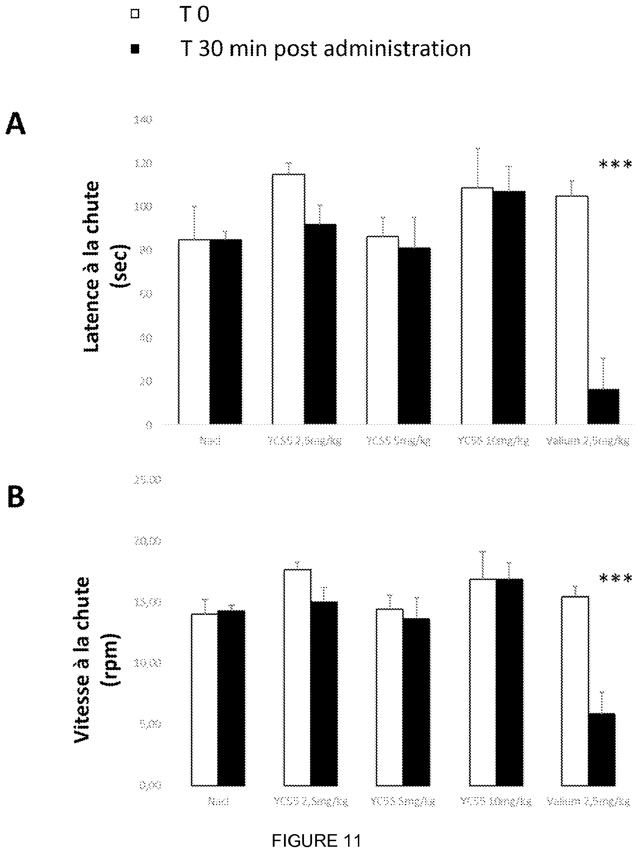

La figure 11 représente des histogrammes montrant la latence à la chute (A) et à la vitesse de chute (B) de souris traitées avec le véhicule, le YC55 (2.5, 5 ou 10 mg/kg, s.c.) ou le valium (2.5 mg/kg, s.c.) soumise à un test de rotarod à vitesse croissante (de 4 à 40 tours/minute pendant 10 min). *** p<0.001 T30 min post administration (■) vs T0 (□) pour une molécule donnée, t-test de Student.

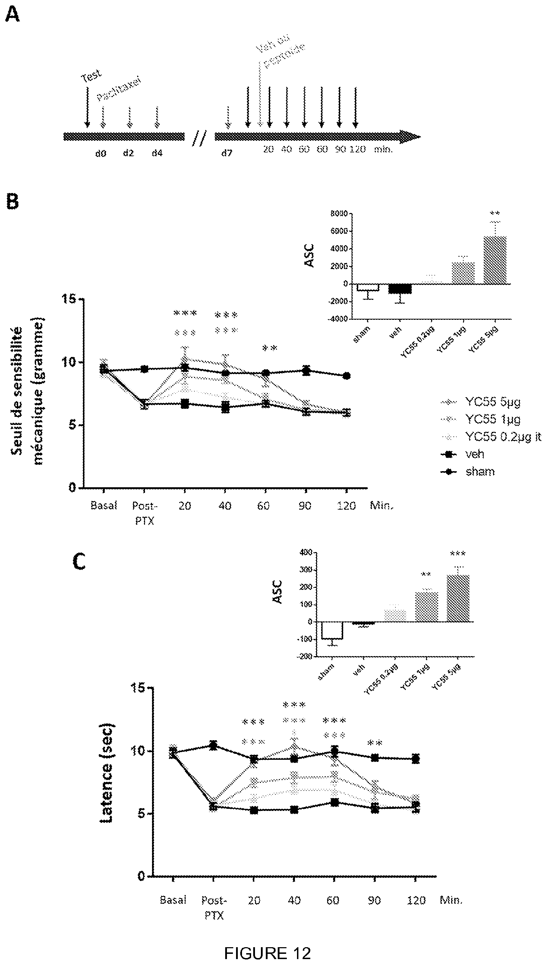

La figure 12 représente (A) conception de l'étude (B) Evolution temporelle de la moyenne +/- SEM des seuils mécaniques (en g) et du seuil de latence à l'immersion de la patte (en sec) et (C) aire sous la courbe (ASC, sec.min) de rats PTX (n=7-9) traités avec le véhicule ou le peptoïde YC55 (0.2, 1 ou 5 µg, i.t.) ; * p<0.05, ** p<0.01, *** p<0.001, vs le groupe veh., ANOVA à deux voies et test *post hoc* de Tukey.

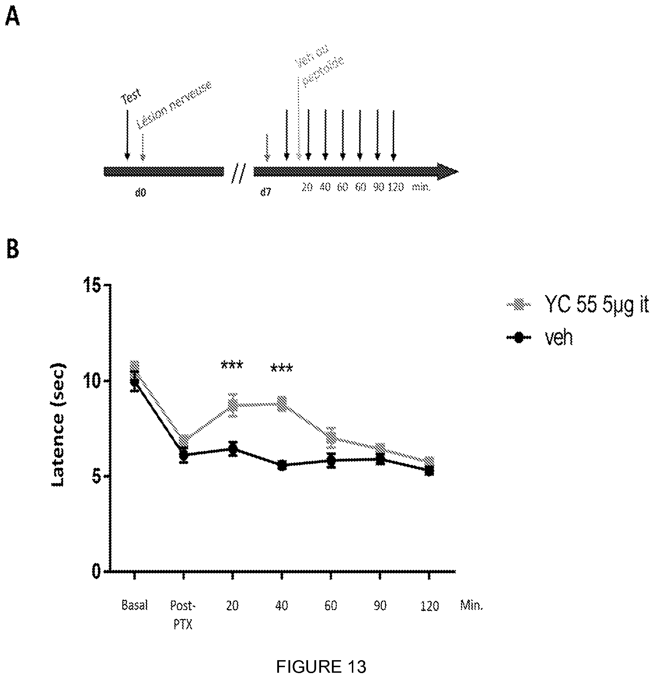

La figure 13 représente (A) conception de l'étude (B) Evolution temporelle de la moyenne +/- SEM du seuil de latence à l'immersion de la patte (en sec) de rats neuropathiques (n=7-9) traités avec le véhicule (●) ou le peptoïde YC55 ( 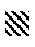 ) (5 µg, i.t.) ; *** p<0.001, vs le groupe veh., ANOVA à deux voies et test *post hoc* de Tukey.

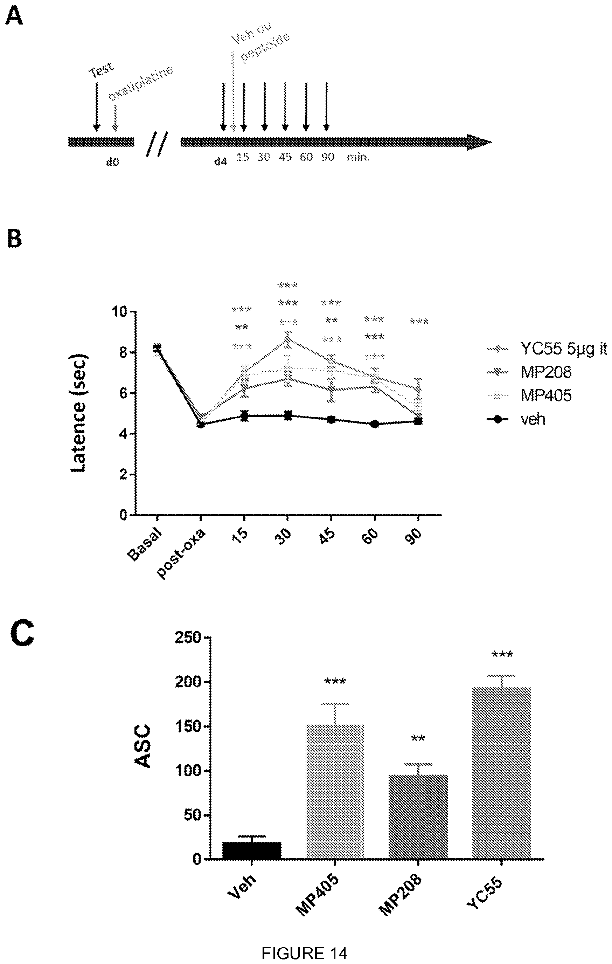

La figure 14 représente (A) conception de l'étude (B) Evolution temporelle de la moyenne +/- SEM du seuil de latence à l'immersion de la patte (en sec) et (C) aire sous la courbe (ASC, sec.min) de l'effet analgésique de souris OIPN (n=7-9) traitées avec le véhicule ou les peptoïdes MP208, MP405, ou YC55 (5 µg, i.t.) ; * p<0.05, ** p<0.01, *** p<0.001, vs le groupe veh., ANOVA à deux voies et test *post hoc* de Tukey.

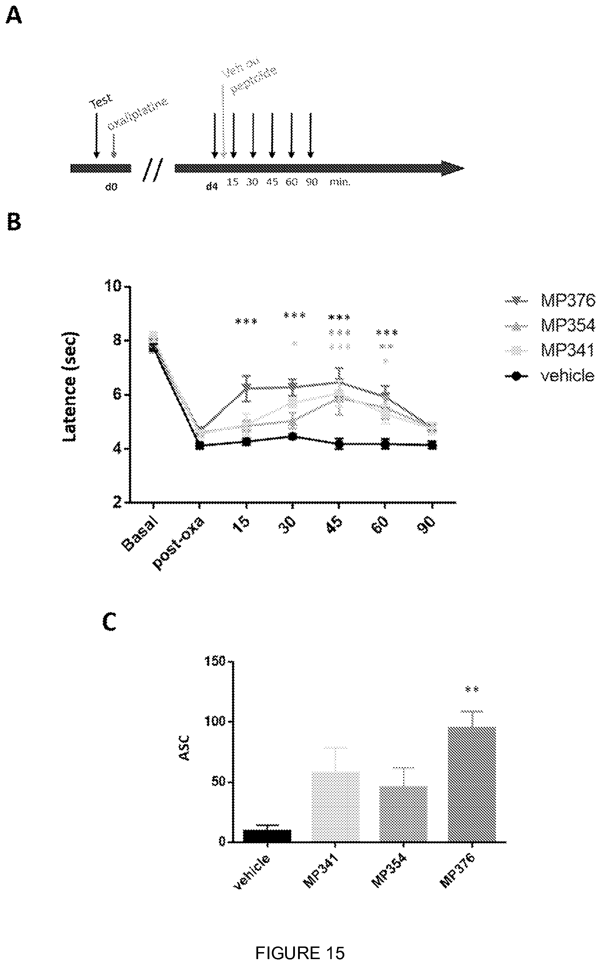

La figure 15 représente (A) conception de l'étude (B) Evolution temporelle de la moyenne +/- SEM du seuil de latence à l'immersion de la patte (en sec) et (C) aire sous la courbe (ASC, sec.min) de l'effet analgésique de souris OIPN (n=7-9) traitées avec le véhicule ou les peptoïdes MP376, MP354, ou MP341 (10 mg/kg, s.c.) ; * p<0.05, ** p<0.01, *** p<0.001, vs le groupe veh., ANOVA à deux voies et test *post hoc* de Tukey.

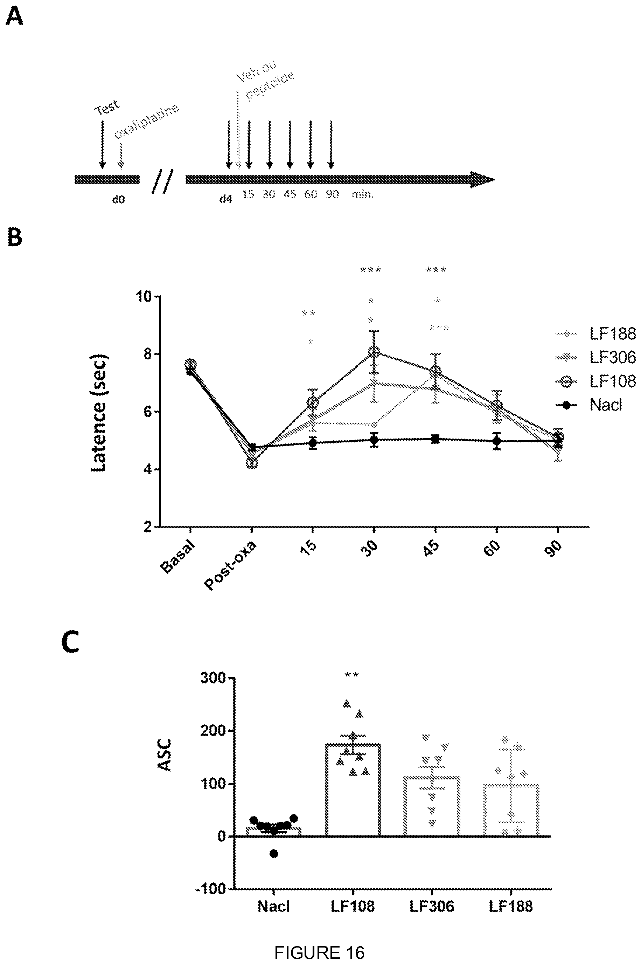

La figure 16 représente (A) conception de l'étude (B) Evolution temporelle de la moyenne +/- SEM du seuil de latence à l'immersion de la patte (en sec) et (C) aire sous la courbe (ASC, sec.min) de l'effet analgésique de souris OIPN (n=7-9) traitées avec le véhicule ou les peptoïdes LF108, LF306, ou LF188 (5 µg, i.t.) ; * p<0.05, ** p<0.01, *** p<0.001, *vs* le groupe veh., ANOVA à deux voies et test *post hoc* de Tukey.

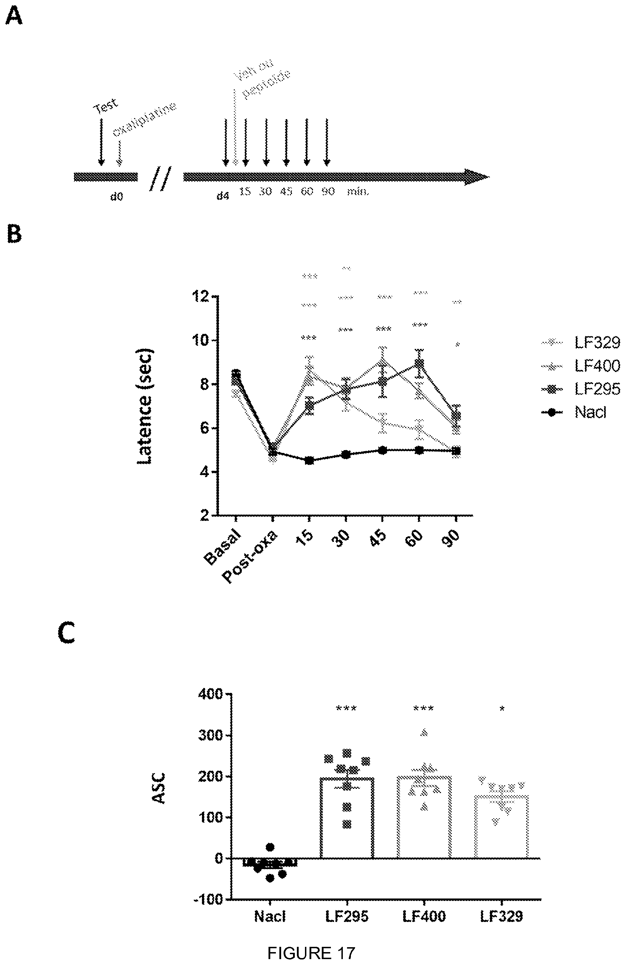

La figure 17 représente (A) conception de l'étude (B) Evolution temporelle de la moyenne +/- SEM du seuil de latence à l'immersion de la patte (en sec) et (C) aire sous la courbe (ASC, sec.min) de l'effet analgésique de souris OIPN (n=7-9) traitées avec le véhicule ou les peptoïdes LF295, LF400, ou LF329 (5 µg, i.t.) ; * p<0.05, ** p<0.01, *** p<0.001, vs le groupe veh., ANOVA à deux voies et test post hoc de Tukey.

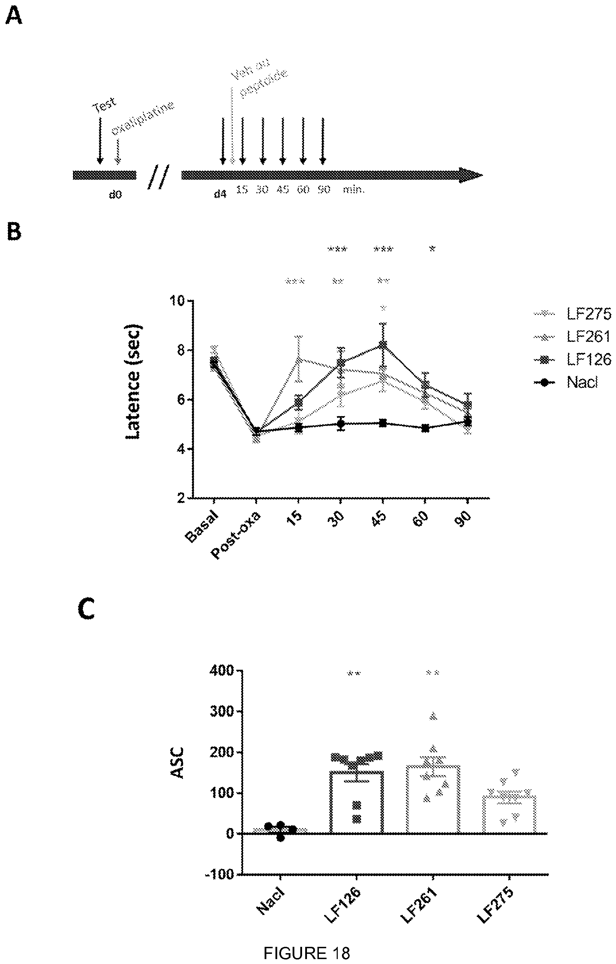

La figure 18 représente (A) conception de l'étude (B) Evolution temporelle de la moyenne +/- SEM du seuil de latence à l'immersion de la patte (en sec) et (C) aire sous la courbe (ASC, sec.min) de l'effet analgésique de souris OIPN (n=7-9) traitées avec le véhicule ou les peptoïdes LF126, LF261, ou LF275 (5 µg, i.t.) ; * p<0.05, ** p<0.01, *** p<0.001, vs le groupe veh., ANOVA à deux voies et test post hoc de Tukey.

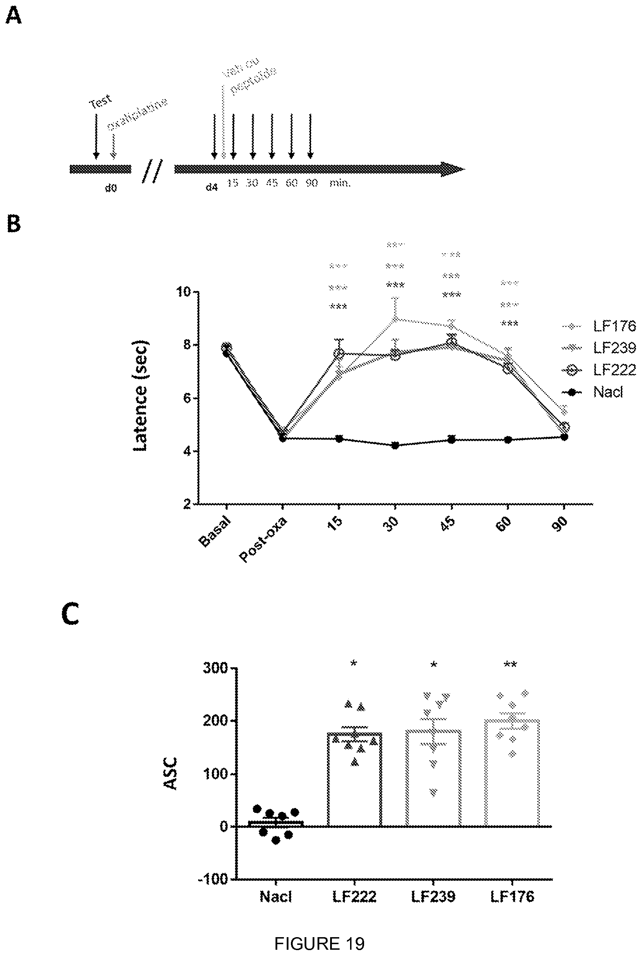

La figure 19 représente (A) conception de l'étude (B) Evolution temporelle de la moyenne +/- SEM du seuil de latence à l'immersion de la patte (en sec) et (C) aire sous la courbe (ASC, sec.min) de l'effet analgésique de souris OIPN (n=7-9) traitées avec le véhicule ou les peptoïdes LF222, LF239, ou LF176 (5 µg, i.t.) ; * p<0.05, ** p<0.01, *** p<0.001, vs le groupe veh., ANOVA à deux voies et test *post hoc* de Tukey.

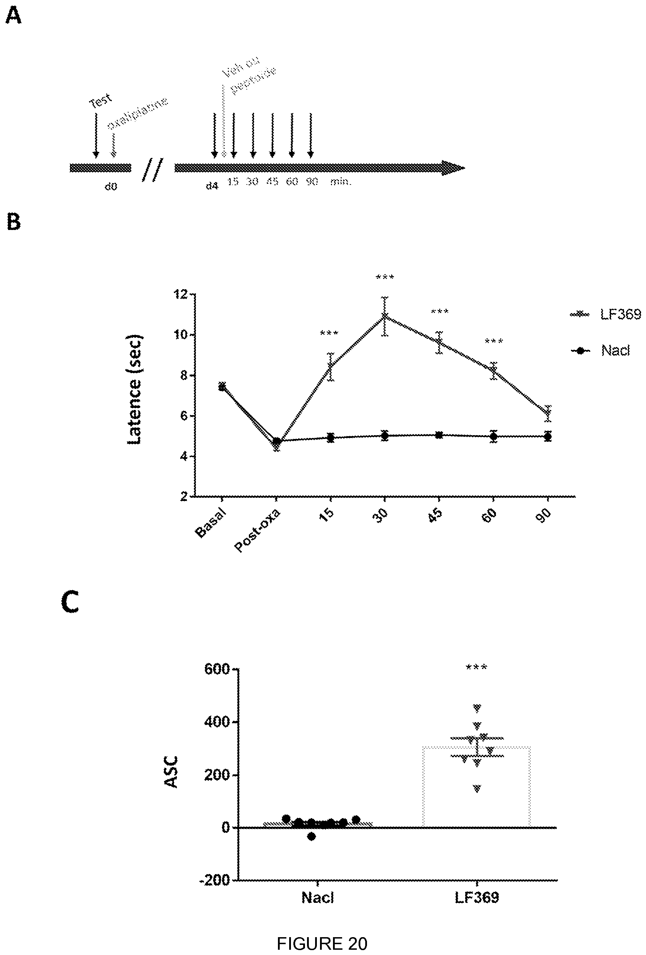

La figure 20 représente (A) conception de l'étude (B) Evolution temporelle de la moyenne +/- SEM du seuil de latence à l'immersion de la patte (en sec) et (C) aire sous la courbe (ASC, sec.min) de l'effet analgésique de souris OIPN (n=7-9) traitées avec le véhicule ou le peptoïde LF369 (5 µg, i.t.) ; * p<0.05, ** p<0.01, *** p<0.001, vs le groupe veh., ANOVA à deux voies et test *post hoc* de Tukey.

### EXEMPLES

### EXEMPLE 1: MATERIEL ET METHODES

### Animaux

Les expériences ont été réalisées sur des souris mâles C57BI/6JRj de 20 à 24 g ou des rats Sprague-Dawley mâles de 150 à 175 g fournis par les laboratoires Janvier (France), maintenus à un cycle lumière/obscurité de 12 heures et nourris et abreuvés *ad libitum.* Les expériences comportementales ont été réalisées à l'aveugle, dans une pièce calme, par le même expérimentateur pour un test donné en prenant grand soin de minimiser ou d'éviter l'inconfort des animaux. Toutes les procédures sur les animaux ont été approuvées par le comité local d'éthique animale et les expériences ont été réalisées conformément aux lignes directrices fournies par la Communauté européenne concernant les soins et l'utilisation des animaux (directive 2010/63/UE).

### Test comportementaux

*Test de Von Frey :* La sensibilité mécanique a été évaluée avec des filaments de Von Frey calibrés de 0,07 g, 0,6 g ou 1,4 g. Les filaments ont été appliqués cinq fois dans l'ordre de rigidité croissante, perpendiculairement à la surface plantaire de la patte arrière et pressés jusqu'à ce qu'ils se plient. Le nombre de réponses pour une force de filament donnée a été compté.

*Test d'immersion* : La queue ou la patte a été immergée dans un bain d'eau réglé à 10°C ou 46°C jusqu'à ce qu'un retrait soit observé (temps de coupure : 30 s). La moyenne de deux déterminations distinctes de la latence du retrait a été calculée (Janssen, Niemegeers & Dony, 1963) [14].

*Test de pression des pattes chez le rat* : Les rats ont été soumis à l'épreuve de pression des pattes décrite précédemment par Randall et Selitto (1957) [15]. Les seuils nociceptifs, exprimés en grammes, ont été mesurés avec un analgésimètre Ugo Basile (Apelex, diamètre de la pointe de la sonde 1 mm, poids 30 g) en appliquant une pression croissante sur la patte arrière droite des rats jusqu'à l'obtention d'un signe de douleur (seuil de vocalisation) (le cut-off a été de 750 g). Avant les traitements, les rats ont été habitués au test en les manipulant sans les soumettre à la pression de la patte. Ensuite, après avoir obtenu deux valeurs de seuil de vocalisation stables consécutives, les effets du traitement ont été évalués après 15, 30, 45, 60, 90 et 120 min. Les résultats sont exprimés sous forme de seuils de vocalisation, en grammes. Pour étudier les effets globaux, les aires sous les courbes d'évolution dans le temps (ASC, g.min) des effets antihyperalgésiques ont été calculées à partir des scores individuels à chaque fois, en utilisant la méthode trapézoïdale. Les données ont été analysées par un ANOVA à deux voies suivi d'un test de Bonferroni, lorsque l'évolution temporelle des effets a été étudiée. Un ANOVA à une voie suivi d'un test de Student Newman-Keuls ont été utilisés pour analyser l'effet des différents traitements déterminés par les ASC. Le niveau de signification statistique a été fixé à p < 0,05.

*Test du Rotarod* : Pour le test du Rotarod (Bioseb), les animaux ont d'abord été habitués à rester sur le cylindre immobile pendant 10 min avant de passer à 10 autres min avec une vitesse de rotation de 4 tours par minute. Les animaux ont reçu le médicament immédiatement après l'entraînement et le test a été effectué 30 minutes plus tard. Le test a consisté à mesurer la latence à la chute des animaux lorsque la rotation passe de 4 à 40 tr/min de façon linéaire sur 5 min. La moyenne des deux valeurs les plus proches sur 3 essais a été calculée.

*Enregistrement de la fréquence cardiaque in vivo* : Les enregistrements télémétriques de l'ECG ont été réalisés à l'aide d'émetteurs télémétriques TA10EA-F20 (DSI) dans une poche sous-cutanée avec des électrodes filaires jumelées placées sur le thorax comme publié précédemment (Mesirca et al., 2014) [27].

### Modèle de douleur animale

*Neuropathie aiguë induite par l'oxaliplatine chez la souris* : Les souris ont reçu une injection intrapéritonéale (i.p.) d'oxaliplatine à 6 mg/kg dissous dans du glucose à 5% directement après la première évaluation comportementale. 90h après l'administration du médicament, correspondant au temps nécessaire pour atteindre le pic d'hyperalgésie (Descoeur et al., 2011) [9], une deuxième évaluation de la sensibilité au froid et de la sensibilité mécanique a été réalisée.

*Neuropathie aiguë induite par le paclitaxel chez le rat :* Les rats ont reçu une injection i.p. de diméthyl sulfoxyde (DMSO ; solvant) ou de 1 mg/kg/1 mL de paclitaxel (Sigma-Aldrich, Lyon, France) aux jours J0, J2, J4 et J7. Le test de comportement a été réalisé le jour de la première injection de paclitaxel (J0) et aux jours 10 (J10) et 14 (J14) après l'injection de paclitaxel.

*La ligature des nerfs spinaux a provoqué une neuropathie chez le rat* : Selon la procédure de Kim et Chung (1992) [16], les rats ont été placés en position couchée et anesthésiés avec 10 mg/kg de xylazine et 75 mg/kg de kétamine (i.p). La fourrure du côté gauche de la colonne vertébrale a ensuite été rasée pour exposer la peau. La peau a été coupée parallèlement à la colonne vertébrale et les muscles paravertébraux ont été séparés. L'apophyse transverse L5 a été enlevée jusqu'à ce que le nerf spinal L5 soit exposé. Le nerf L5 a été isolé et ligaturé avec du fil de soie. Enfin, les incisions musculaires et cutanées ont été suturées et la plaie a été désinfectée avec de l'iodophore et de l'alcool éthylique à 75%. Dans le groupe sham, les rats ont subi la même procédure chirurgicale sans la ligature du nerf.

### Tests ex vivo

*Isolement et quantification de l'ARN* : Les paires de DRG des segments lombaires L4 à L6 ont été disséquées et mises en commun. L'ARN a été extrait à l'aide du réactif TRIzol^{®} (Azote) conformément aux instructions du fabricant (Thermofisher). La qualité de l'ARN a été évaluée par le bioanalyseur Agilent 2100 (Agilent Technologies), les échantillons dont le RIN (RNA Intégrity Number) était supérieur à 8 ont été conservés pour la suite des analyses. La transcription inverse a été effectuée avec la transcriptase inverse SuperScript II conformément au protocole fourni par Invitrogen. Pour la PCR quantitative en temps réel, l'amplification a été réalisée en utilisant le kit Light Cycler Fast Start DNA (Roche diagnostic) et la quantité d'ADN en temps réel a été mesurée avec un thermocycleur Mastercycler Realplex (Eppendorf). La moyenne de la quantité relative d'ADNc de chaque triplicat a été calculée à l'aide d'une courbe de concentration standard et a été normalisée sur l'ADNc du gène GUS. Les conditions d'amplification étaient les suivantes : préincubation pendant 7 min à 95 °C suivi de 50 cycles de 20 s à 95 °C et 20 s à la température spécifique d'hybridation de la paire d'amorces suivi de 20 s à 72°C.

*Séquençage de l'ARN* : Nous avons utilisé l'analyse du séquençage de l'ARN sur les DRG L4-L5-L6 de souris traitées à l'oxaliplatine (3mg/kg, i.p.) ou au véhicule deux fois par semaine pendant 3 semaines. À la fin de l'expérience (D21), les souris ont été anesthésiées et mises à mort, et les DRG L4-L5-L6 ont été rapidement récoltés, congelés dans de l'azote liquide et stockées à -80°C jusqu'à leur utilisation. Les ARNs totaux des DRG L4-L5-L6 ont été extrait à l'aide du kit RNeasy Micro (Qiagen) selon le protocole du fabricant et l'ARN Ribosomal éliminé en utilisant Ribo-Zero Plus rRNA depletion kit (Illumina). La concentration d'ARN a été déterminée à l'aide de la plaque de quantification des acides nucléiques Take3 (Epoch, BioTek, USA). L'intégrité de l'ARN a été analysée par un instrument d'électrophorèse capillaire (Fragment Analyser, Agilent^{®}) et envoyée à Fasteris (https://www.fasteris.com) pour le séquençage de l'ARN. Les bibliothèques ont été préparées en utilisant le protocole TruSeq d'Illumina et un séquençage basé sur SBS a été réalisé en utilisant une plateforme HiSeq 2500 (Illumina). L'analyse a été effectuée en différentes étapes : cartographie des jonctions d'épissage (TopHat2), comptage (HTSeq-count), filtrage, normalisation (edgeR, DESeq et DESeq2) et analyse différentielle (edgeR, DESeq et DESeq2) effectuée par Benjamin Bertin et Yoan Renaud (GreD, Clermont-Ferrand).

*Fractionnement des membranes* : Les tissus ont été homogénéisés dans un tampon HEPES (20mM HEPES pH 7,4, 320mM de saccharose, 5mM d'EDTA, 5mM d'EGTA) contenant des inhibiteurs de protéases à 4 °C, soniqués et centrifugés à 1000 g pendant 15min à 4°C. Les culots ont été éliminés. Pour le spin des organelles, les surnageants ont été centrifugés à 15 000 g pendant 10 min à 4°C et les culots d'organelles ont été conservés. Pour le spin cytosolique, les surnageants ont été centrifugés à 100 000 g pendant 1 heure à 4°C. La fraction cytosolique se trouvait dans les surnageants et la fraction membranaire dans les culots. Les culots de membranes ont ensuite été remis en suspension dans le tampon SB (20mM HEPES pH 7,4, 1M KI, 5mM EDTA, 5mM EGTA à 4 degrés) et centrifugé à 100 000 g pendant 1 heure à 4°C pour éliminer les protéines membranaires faiblement liées. Les fractions membranaires ont ensuite été remises en suspension dans du tampon RB (20mM HEPES pH 7,4, 100mM NaCl, 5mM EDTA, 5mM EGTA) contenant du Triton (1 %) et des inhibiteurs de protéases. Après sonication dans un bain de glace, les fractions membranaires ont par la suite été soumises à une agitation douce pendant 2 heures à 4°C. °C. Les fractions membranaires solubilisées ont été centrifugées à 100 000 g pendant 1 heure à 4°C. La fraction membranaire se trouvait dans le surnageant. *Western blot* : Les DRG L4 à L6 de trois souris ont été mis en commun et homogénéisés dans un tampon de lyse contenant du triton (1%) et des inhibiteurs de protéases. Les homogénats ont été centrifugés à 18 000 g 20 min à 4°C et les surnageants collectés. 50 µg de protéines totales ont été séparées par SDS-PAGE sur un gel à 7,5 %. Après transfert sur membrane de nitrocellulose et saturation avec du BSA (5%), les anticorps primaires ont été utilisés à une dilution de 1/500 pour HCN1 et HCN2 (Neuromab), 1/1000 pour TRIP8b (Neuromab), et 1/5000 pour Actinβ (Sigma-Aldrich). Après 3 lavages successifs avec du TBST, les anticorps secondaires couplés HRP correspondants (ThermoFisher) ont été incubés à une dilution au 1/10 000 et détectés avec le substrat chimioluminescent (SuperSignal West Pico Chemiluminescent Substrate, Thermofisher).

*Enregistrement par patch-clamp* : Les expériences d'électrophysiologie ont été réalisées sur des cellules cardiaques pacemaker isolées comme précédemment décrit (Marger et al., 2011) [28]. La technique du patch-clamp en cellule entière a été utilisée pour enregistrer les courants If générés par les canaux HCN, en employant un amplificateur Axopatch 200A (Axon Instruments Inc., Foster USA). Les électrodes d'enregistrement de résistance de -5m MΩ ont été remplies par une solution intracellulaire contenant (mM/L) : K+-aspartate, 130 ; NaCl, 10 ; sel ATP-Na+, 2 ; phosphate de créatine, 6,6 ; GTP-Mg2+, 0,1 ; CaCl2, 0,04 (pCa = 7) ; Hepes-KOH, 10 ; (pH = 7,2 avec du KOH). Une solution extracellulaire de Tyrode contenant 5 mM de BaCl2 pour bloquer les courants IK1 a été utilisée. L'effet de la molécule NUCC5953 (5µM) sur l'amplitude et la cinétique du courant If a été comparé à celui de l'ivabradine (3µM), tous les deux dilués dans la solution extracellulaire. L'acquisition des données a été effectuée en utilisant le logiciel pClamp (ver. 9, Axon Instruments Inc.).

### Essais in vitro

*Essai de polarisation de la fluorescence* : un test basé sur la polarisation de fluorescence (FP) a été utilisé pour identifier les composés capables de perturber l'interaction entre le HCN et la queue TRIP8b, comme cela a été publié (Han et al., 2015) [13].

### EXAMPLE 2 : RESULTATS

Pour la première fois la présence de l'ARNm TRIP8b (les données RNAseq obtenues à partir de souris DRG montrent la présence (FPKM>1) des variantes d'épissage 2 à 5 : NM_01163516, NM_01163517, NM_01289505, NM_01310460) et de la protéine dans les neurones DRG et TG chez la souris, a été observée. De plus, l'expression des transcrits et des protéines des canaux HCN1-2 et de TRIP8b a été significativement augmentée dans les neurones périphériques des animaux traités à l'oxaliplatine par rapport aux témoins, ce qui suggère l'implication des 2 protéines dans le développement de l'OIPN (souris modèle de neuropathie induite par oxaliplatine) (Figure 1 A-C). L'analyse de l'expression de la protéine TRIP8b dans la moelle épinière des animaux traités avec une seule (Figure 1D) ou après administration répétée (figure 1E) d'oxaliplatine par rapport au véhicule montre une nette augmentation de la protéine TRIP8b sous l'effet de l'oxaliplatine.

En utilisant NUCC5953, une molécule capable de perturber l'interaction TRIP8b-HCN (Han et al., 2015) [13], il a été démontré que l'hypersensibilité au froid aiguë induite par l'oxaliplatine peut être inversée en fonction de la dose administrée soit par voie i.t. (0,2 ; 1 ; 5 µg /souris ; Figure 2) ou par voie systémique (4mg/kg ; Figure 3).

Aucune altération de l'activité locomotrice n'a été observée lors du test Rotarod lorsque les souris ont été traitées avec NUCC5953 (4mg/kg) administrée par voie sous-cutanée (Figure 4).

Parallèlement, des données préliminaires suggèrent également que l'administration de NUC5953 réduit la surexpression des protéines HCN1 et 2 et TRIP8b dans la fraction membranaire des neurones de DRG des animaux OIPN (Figure 5).

Les expériences de patch-clamp sur des cellules pacemaker cardiaque isolées n'ont montré aucun effet du NUCC5953 sur l'amplitude du courant If, contrairement à l'Ivabradine, un bloqueur pan-HCN (Figure 6).

NUCC5953 n'affecte pas la fréquence cardiaque *in vivo* lorsqu'il a été administré à une dose analgésique (4mg/kg, i.p.) (Figure 7). Les résultats montrent que la moyenne de fréquence cardiaque n'est pas modifiée chez trois souris avant (A), au moment de l'injection de NUCC5953 (B) et 30 minutes après injection (C).

### EXEMPLE 3 : CONCEPTION DES PEPTIDOMIMETIQUES

### Conception de peptidomimétiques

### A- Peptidomimétiques tétramères

Des composés peptoïdes imitant la séquence de l'extrémité carboxy-terminale du canal HCN2 ont été conçus et synthétisés. La conception de ces peptidomimétiques s'est appuyée sur une étude approfondie de la structure aux rayons X d'un co-cristal de TRIP8b avec la région C-terminale de HCN2, disponible dans la base de données des protéines (pdb : 4EQF). Un travail de modélisation préparatoire destiné à compléter et à améliorer cette structure a été réalisé. Les parties manquantes ont été modélisées par homologie de séquences provenant de différentes structures PEX5 (PDB : 1FCH, 3R9A, 4KXK, 4KYO, 2J9Q, 2C0M et 2C0L) présentant une identité de séquence allant de 30 à 60% avec TRIP8b. Le modèle résultant a permis d'identifier les contacts entre résidus responsables de la formation du complexe HCN-TRIP8b. Cette étude a notamment permis d'identifier deux poches hydrophobes du domaine TPR de TRIP8b, essentielles à une bonne affinité des ligands à ce domaine protéique (Figure 8).

La poche hydrophobe 1 de TRIP8b assure un ancrage de la chaîne latérale du résidu C-terminal des ligands. Une interaction efficace des ligands avec la poche hydrophobe 2, située à proximité de la région centrale de TRIP8b, identifiée comme site de liaison au domaine CNBD de HCN, permet également de renforcer l'affinité des inhibiteurs. Plusieurs oligomères hybrides peptide-peptoïde de 4 résidus et portant diverses chaînes latérales capables de cibler ces poches hydrophobes ont été conçus et évalués par dynamique moléculaire (DM).

La conception basée sur la structure a permis d'identifier des ligands peptidomimétiques interagissant au site de liaison peptidique SNL (domaines TPR) de TRIP8b. Ces ligands ont une formule qui est représentée par la formule LF108 ci-dessus ou la formule générale (I) suivante : 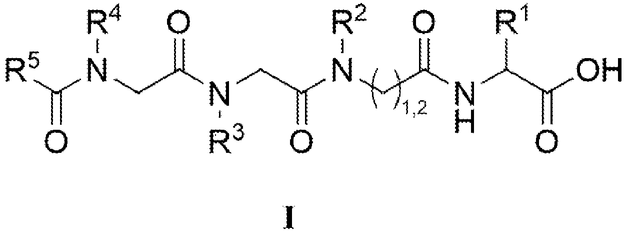 où
**R¹, R², R³, R⁴** et **R⁵** sont tels que définis précédemment.

Les composés sont des oligomères hybrides peptide-peptoïde (peptomères) (Ostergaard et al., 1997) [17]. Ils comprennent quatre résidus dont un α-amino acide positionné à l'extrémité C-terminale de la séquence, et trois résidus peptoïdes consécutifs. Le troisième résidu (à partir de l'extrémité N-terminale) est soit une unité β-peptoïde soit une unité α-peptoïde. Les α-peptoïdes généralement appelés « peptoïdes » font référence aux oligomères de glycine N-substitués (Simon et al., 1992) [18]. Par rapport aux peptides, les chaînes latérales sont attachées aux atomes d'azote des liens amide plutôt qu'aux atomes de carbone α. Les β-peptoïdes sont des oligomères composés d'unités d'acide β-aminopropionique N-substitués (Hamper et al., 1998) [19]. Ainsi, un résidu β-peptoïde contient un groupe méthylène supplémentaire dans le squelette par rapport à un résidu α-peptoïde. Les peptoïdes présentent des caractéristiques avantageuses pour le développement de composés bioactifs. Ils se prêtent à la synthèse en phase solide, en utilisant une approche itérative dite "sous-monomère" dans laquelle chaque nouvelle unité est construite en deux étapes (Zuckermann et al., 1992) [20]. Les peptoïdes peuvent incorporer des chaînes latérales très diverses, aussi bien des chaînes latérales protéinogènes que des chaînes latérales non naturelles (Culf et al., 2010) [21]. Les synthèses « sous-monomères » utilisent des molécules de départ simples telles que l'acide bromoacétique et des amines primaires pour la construction d'unités α et le chlorure d'acryloyle et des amines primaires pour la construction d'unités β-peptoïdes. Les amines primaires, à partir desquelles les chaînes latérales sont incorporées, sont disponibles commercialement par centaines, ou peuvent être préparées avant leur utilisation le cas échéant. Il a été démontré que le squelette peptoïde reposant sur des amides *N,N-*disubstitués (amides tertiaires) est résistant aux protéases (Miller et al., 1995) [22]. La présence de liaisons amides tertiaires leur confère également une meilleure perméabilité cellulaire (Kwon et al., 2007 ; Vollrath et al., 2013) [23, 24], et quelques études tendent à démontrer que les peptoïdes sont moins immunogènes que les peptides (Li et al., 2010 ; Chongsiriwatana et al., 2008) [25, 26].

Les synthèses ont été réalisées sur de la résine chlorure de 2-chlorotrityle (2-CTC) conduisant à un acide carboxylique à l'extrémité C-terminale du peptomère après clivage. Le premier résidu immobilisé sur le support, un α-amino acide, a été choisi principalement dans le groupe des acides aminés protéinogènes hydrophobes, dont la L-leucine qui correspond à l'extrémité C-terminale du peptide SNL. Les α-amino acides ont été immobilisés sur la résine sous une forme protégée N-Fmoc. Après élimination du groupe Fmoc, l'extension de la chaîne par les résidus peptoïdes a été réalisée par des approches de "chimie sous-monomères". Des protocoles connus ont été mis en oeuvre mais certains ajustements techniques ont été nécessaires pour la synthèse du monomère β-peptoïde. L'extrémité N-terminale a été acylée avant le clivage en condition acide par l'hexafluoroisopropanol (HFIP) et la déprotection des chaînes latérales par de l'acide trifluoroacétique (TFA) dans le dichlorométhane

Les composés peptomères bruts ont été analysés par LC-MS puis purifiés sur des cartouches de chromatographie flash C18 avec une détection UV à 214 et 220 nm. Les fractions pures ont été rassemblées et lyophilisés. Les composés purs ont été analysés par HPLC en phase inverse sur colonne Nucleodur^{®} C₄ (5 µm, 300 Å, 250 mm × 4,6 mm). Le Tableau 2 ci-dessous présente les données HRMS et HPLC des composés.

**Tableau 2**

| Référence molécule | masse attendue | masse observée / formule | temps de rétention (min) chromatogrammes LCMS^{a} ou HPLC^{b} | pureté HPLC (%)^{c} |
|---|---|---|---|---|
| YC55 | 564.3159 | 565.3227 [M+H]⁺ / C₂₈H₄₅N₄O₈ | 22,02^{b} | 97 |
| YC60 | 564.3159 | 565.3229 [M+H]⁺ / C₂₈H₄₅N₄O₈ | 3,74^{a} | n.d. |
| MP208 | 598.3003 | 599.3075 [M+H]⁺ / C₃₁H₄₃N₄O₈ | 22,86^{b} | 92 |
| MP281 | 536.2846 | 537.2919 [M+H]⁺ / C₂₆H₄₁N₄O₈ | 20,69^{b} | 96 |
| MP341 | 578.3316 | 579.3388 [M+H]⁺ / C₂₉H₄₇N₄O₈ | 4,05^{a} | n.d. |
| MP354 | 578.3316 | 579.3388 [M+H]⁺ / C₂₉H₄₇N₄O₈ | 3,81^{a} | n.d. |
| MP376 | 578.3316 | 579.3388 [M+H]⁺ / C₂₉H₄₇N₄O₈ | 3,76^{a} | n.d. |
| MP377 | 592.3472 | 593.3545 [M+H]⁺ / C₃₀H₄₉N₄O₈ | 3,81^{a} | n.d. |
| MP405 | 550.3003 | 551.3075 [M+H]⁺ / C₂₇H₄₃N₄O₈ | 21,59^{b} | 85 |
| LF40 | 563.2955 | 564.3021 [M+H]⁺ / C₂₇H₄₂N₅O₈ | 21,66^{b} | 91 |
| LF96 | 594.3265 | 595.3337 [M+H]⁺ / C₂₉H₄₇N₄O₈ | 22,06^{b} | 92 |
| LF108 | 609.3010 | 608.2939 [M+H]⁻ / C₂₈H₄₂N₅O₁₀ | 22,07^{b} | 92 |
| LF176 | 577.3112 | 578.1388 [M+H]⁺ / C₂₈H₄₄N₅O₈ | 21,53^{b} | 97 |
| LF126 | 578.3316 | 579.3381 [M+H]⁺ / C₂₉H₄₇N₄O₈ | 24,53^{b} | 89 |
| LF188 | 632.3033 | 633.3099 [M+H]⁺ / C₂₉H₄₄F₃N₄O₈ | 23,30^{b} | 98 |
| LF222 | 564.3159 | 565.3229 [M+H]⁺ / C₂₈H₄₅N₄O₈ | 21,55^{b} | 94 |
| LF239 | 564.3159 | 565.3228 [M+H]⁺ / C₂₈H₄₅N₄O₈ | 21,12^{b} | 95 |
| LF261 | 582.3065 | 583.3138 [M+H]⁺ / C₂₈H₄₄FN₄O₈ | 21,34^{b} | 98 |
| LF275 | 563.3319 | 564.3395 [M+H]⁺ / C₂₈H₄₆N₅O₇ | 30,79^{b} | 94 |
| LF295 | 550.3003 | 551.3068 [M+H]⁺ / C₂₇H₄₃N₄O₈ | 20,36^{b} | 86 |
| LF306 | 578.3316 | 579.3387 [M+H]⁺ / C₂₉H₄₇N₄O₈ | 21,32^{b} | 95 |
| LF329 | 563.3319 | 564.3383 [M+H]⁺ / C₂₈H₄₆N₅O₇ | 20,90^{b} | 95 |
| LF400 | 578.3316 | 579.3381 [M+H]⁺ / C₂₉H₄₇N₄O₈ | 21,78^{b} | 91 |
| LF369 | 389.4490 | 390.2231 [M+H]⁺ C₁₇H₃₂N₃O₇ | 18,32^{b} | 90 |

| | | | | |
|---|---|---|---|---|
| ^{a}LCMS. Chaîne UHPLC Ultimate 3000 RSLC (ThermoScientific), colonne Kinetex EVO C18 (100 × 2,1mm; 1,7µm) (Phenomenex), débit: 0.45 mL/min, four colonne: 30°C, gradient H₂O (0,1% acide formique/ ACN (0,1% acide formique) : t = 0 min 95:5 ; t = 7,5 min 1:99, t = 8,5 min 1:99 ; t = 9 min 95:5 ; t = 11 min 95:5. ^{b}HPLC Chaîne Agilent série 1100, colonne Nucleodur^{®} C₄ (5 µm, 300 Å, 250 mm × 4,6 mm), débit 0,5 mL/min. gradient H₂O/ACN (0,1% TFA) : t = 0 min 95:5 ; t = 5 min 95:5 ; t = 25 min 5:95 ; t = 35 min 5:95, t = 40 min 95:5, t = 50 min 95:5. ^{c}Détection UV 214 nm | | | | |

### Chaînes latérales

**R¹** est de manière indépendante CH₃ (L-Ala), CH(CH₃)₂ (L-Val), CH(CH₃)CH₂CH₃ (L-Ile), CH₂CH(CH₃)₂ (L-Leu and D-Leu), CH₂C(CH₃)₃, C(CH₃)₃, CH₂(cyclobutyl) ;
**R²**, **R³**, et **R⁴** sont les chaînes latérales des unités peptoïdes incorporées séquentiellement au cours de la synthèse à partir d'amines primaires ;
**R²** et **R³** sont choisis de manière indépendante parmi : CH₂CH(CH₃)₂, CH₂CH₂NH₂, CH₂CH₂OH, CH₂CH₂CH₂OH, CH₂CH(OH)CH₃, CH₂CONH₂, CH₂CH₂CONH₂. Les chaînes latérales présentant une fonction alcool ou amine ont été incorporées aux oligomères sous forme protégée : éther de silyle pour la fonction alcool et tert-butyl carbamate (Boc) pour la fonction amine ;
**R⁴** est un groupe aliphatique ou aromatique choisi parmi : 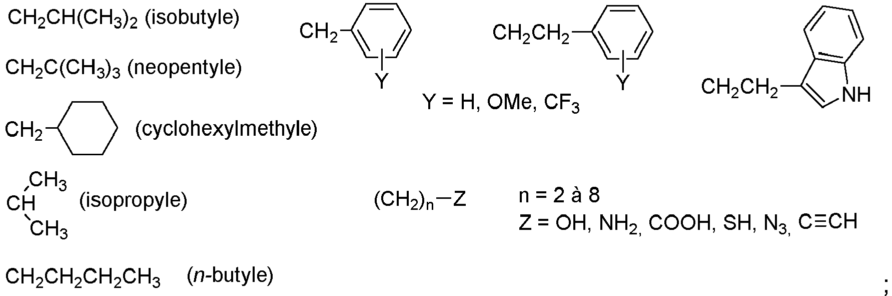
**R**⁵ est de manière indépendante CH₃, CH(CH₃)₂, CH₂C₆H₅, C₆H₄X (où X en position ortho, méta ou para est de manière indépendante H, OMe, CF₃, CH₃, CH₂CH₃, F, Br, Cl, NO₂), 3-indolyl, 3-quinolinyl.

### Schéma synthétique représentatif sur résine de chlorure de 2-chlorotrityle, pour la synthèse de peptomères N-acylés incorporant un acide α-aminé à l'extrémité C-terminale, suivi d'un résidu ß- et de deux résidus α-peptoïdes.

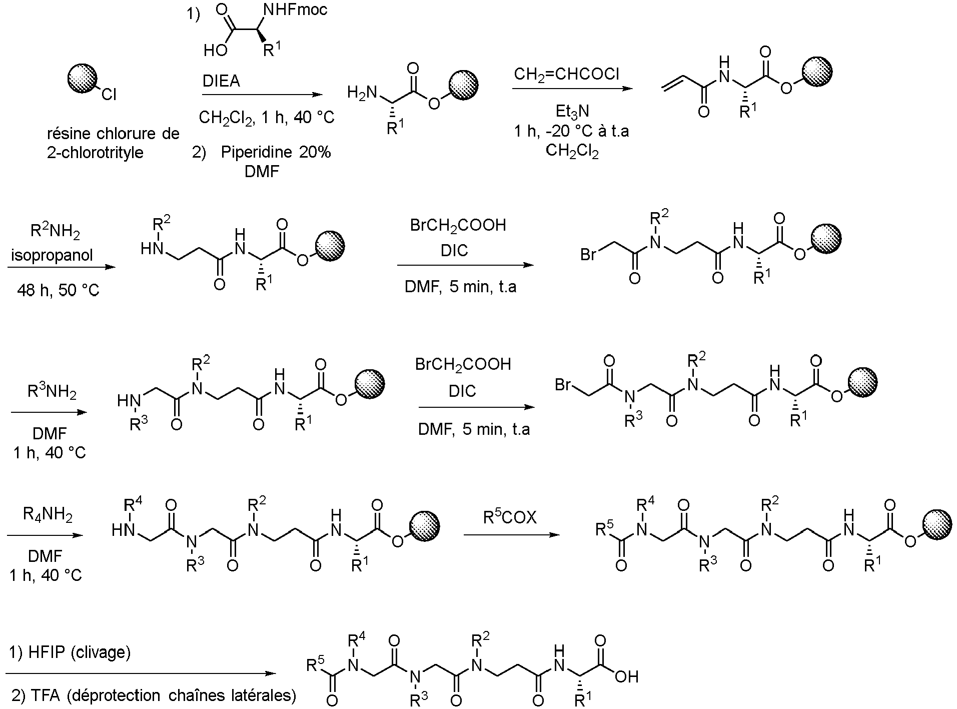

DIEA : diisopropyléthylamine ; Fmoc : fluorénylméthyloxycarbonyle ; DMF : N,N-diméthylformamide ; DIC : diisopropylcarbodiimide ; HFIP : 1,1,1,3,3,3-hexafluoroisopropanol ; t.a : température ambiante ; TFA : acide trifluoroacétique

### Molécules synthétisées

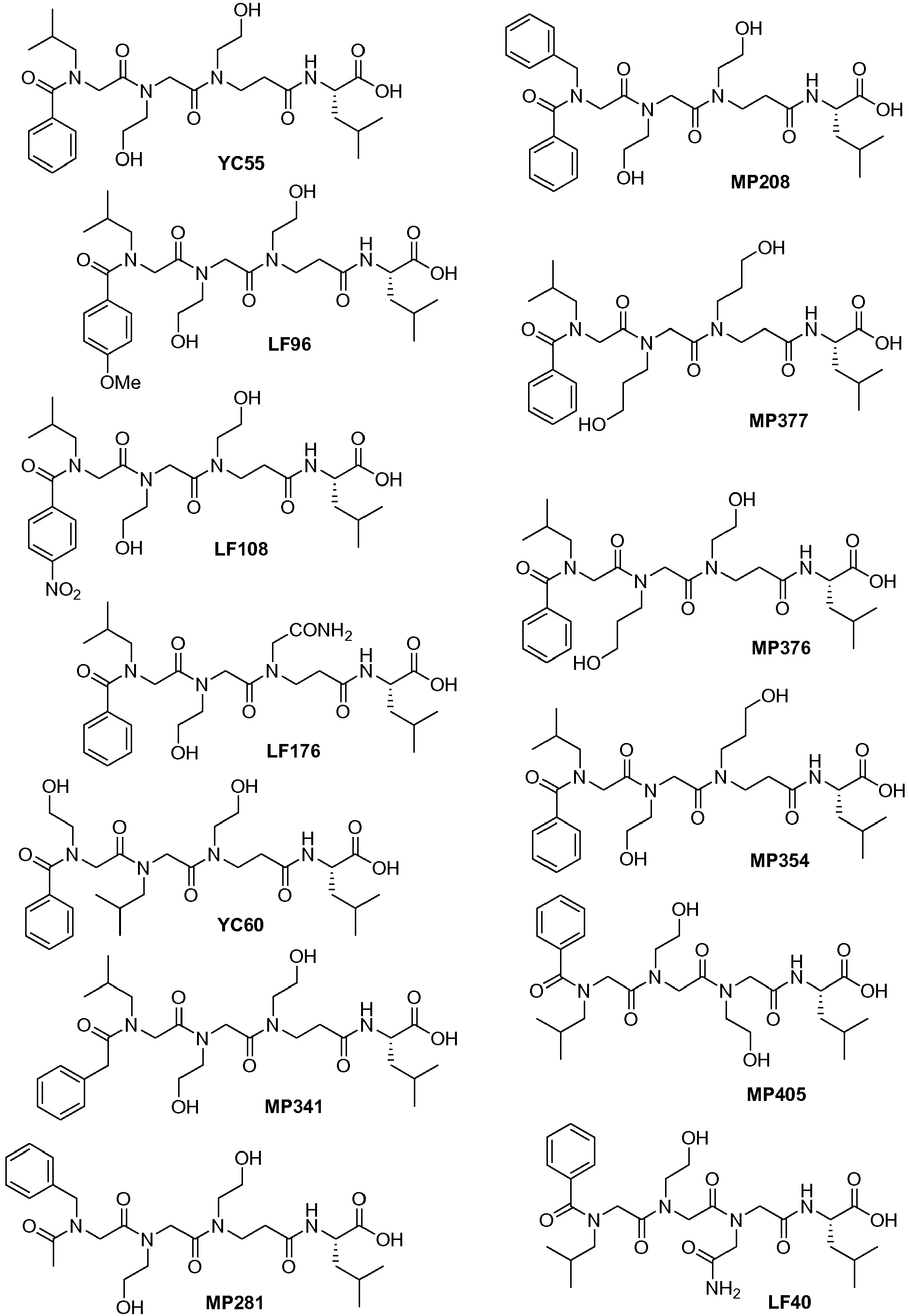

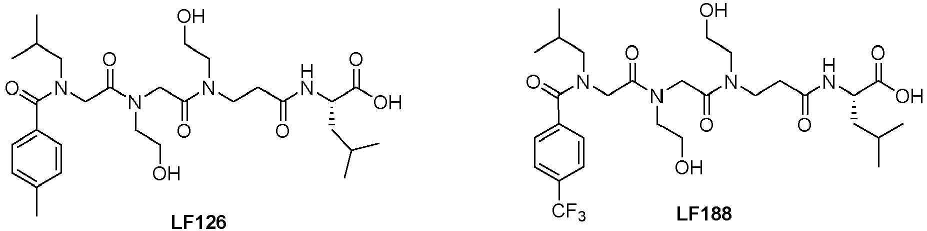

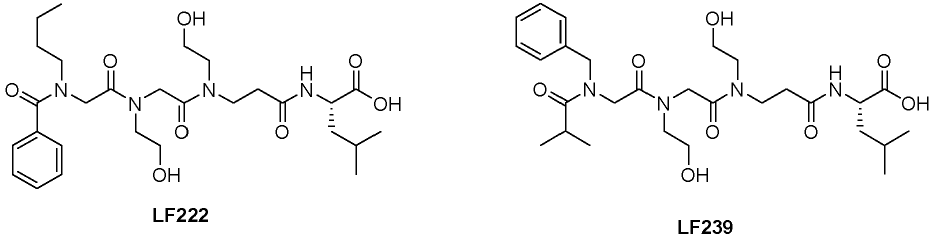

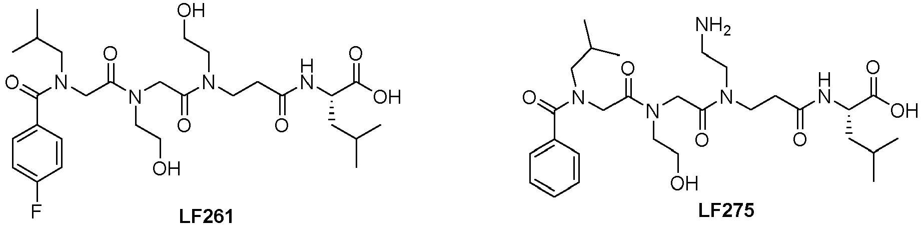

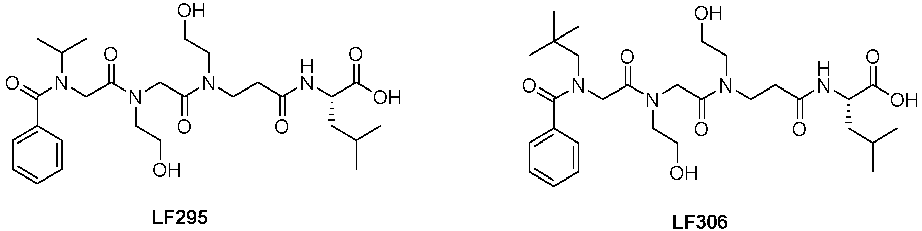

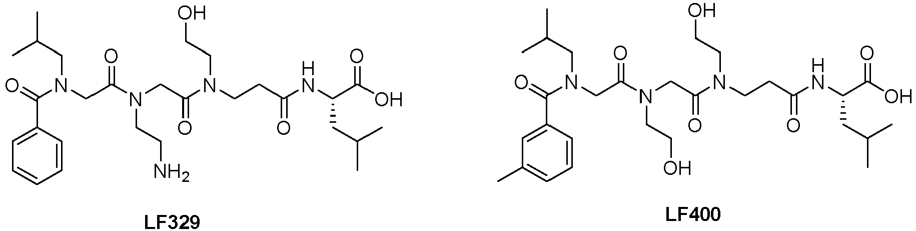

### Synthèse

Les composés correspondant à la formule générale (I) ont été synthétisés sur une résine de type chlorure de 2-chlorotrityle (Novabiochem 100-200 mesh) dans des seringues en plastique de 5 mL équipées d'un fritté et d'un robinet d'arrêt. Les seringues ont été placées sur une plateforme chauffante à agitation orbitale pendant les réactions.

Dans une synthèse typique décrite avec de la L-leucine à l'extrémité C-terminale et un résidu β-peptoïde précédant cet acide aminé, de la résine chlorure de 2-chlorotrityle (1,33 mmol g⁻¹, 0,100 g, 0,133 mmol) a été mise à gonfler dans 2 mL de CH₂Cl₂ sec (2 × 20 min). Après filtration, 0,12 mL d'une solution 2,2 M de Fmoc-Leu-OH dans le CH₂Cl₂ (2,0 équivalents) a été ajoutée, suivie de 2,0 mL d'une solution 0,4 M de DIEA dans le CH₂Cl₂ (6,0 équivalents). Le mélange a été agité pendant 40 min à 25 °C. La résine a été égouttée, lavée avec du CH₂Cl₂ (2 × 2 mL) et la fixation de la Fmoc-Leucine à la résine a été répétée dans les mêmes conditions. La résine a été lavée avec du CH₂Cl₂ (5 × 2 mL). Le groupe Fmoc a été éliminé par ajout de 2,0 mL d'une solution de pipéridine à 20 % dans le DMF et agitation pendant 15 minutes à température ambiante. L'opération a été répétée dans les mêmes conditions, après quoi la résine a été lavée avec du DMF (5 × 2 mL) et égouttée.

Introduction du résidu β-peptoïde. La résine a été refroidie par deux lavages avec du CH₂Cl₂ à -20 °C. 1,21 mL d'une solution 0,22 M de chlorure d'acryloyle (2,0 équivalents) dans du CH₂Cl₂ refroidie à -20°C a été ajoutée, suivie de 0,8 mL d'une solution 0,5 M de Et₃N (3,0 équivalents) dans du CH₂Cl₂ refroidie (-20 °C). La résine a été agitée pendant 1 h à 25 °C, égouttée, puis traitée à nouveau avec du chlorure d'acryloyle dans les mêmes conditions. La résine a été filtrée et lavée avec du CH₂Cl₂ (5 × 2 mL), puis égouttée. L'acrylamide obtenu a été traité par une solution 2,0 M de l'amine R²NH₂ dans l'isopropanol (12,0 équivalents) pendant 24 h à 50 °C. Après filtration de la résine, la réaction d'aza-Michael a été répétée dans les mêmes conditions. La résine a été lavée avec de l'isopropanol (5 × 2 mL) avant la synthèse sous-monomère de deux résidus α-peptoïdes.

Synthèse des résidus α-peptoïdes. A la résine ont été ajoutées 2,0 mL d'une solution 0,4 M d'acide bromoacétique dans le DMF (6,0 équivalents), suivie de 2,13 mL d'une solution 0,5 M de DIC dans le DMF (8,0 équivalents). Après avoir été agitée pendant 5 minutes à 25 °C, la résine a été égouttée et lavée avec du DMF (5 × 2 mL). Le bromoacétamide obtenu a ensuite été traité par une solution 2.0 M de l'amine R³NH₂ (20,0 équiv) dans le DMF et le mélange a été laissé sur la plate-forme d'agitation pendant 1 h à 40 °C. La résine a été filtrée et lavée avec du DMF (5 × 2 mL). Les deux dernières étapes de bromoacétylation et de substitution ont été reproduites dans les mêmes conditions afin de construire le quatrième résidu portant la chaîne latérale R⁴ souhaitée. L'extrémité N-terminale du peptomère a ensuite été acylée selon l'une des procédures générales décrites ci-après, avant son décrochage de la résine.

Pour le clivage, la résine a été traitée par 1,5 mL d'une solution 2,4 M de HFIP dans du CH₂Cl₂. Après agitation pendant 40 minutes à 25°C, la résine a été filtrée et lavée avec du CH₂Cl₂ (5 × 2 mL). Le solvant a été éliminé sous pression réduite. Enfin, les groupes protecteurs silylés ou Boc des chaînes latérales ont été éliminés par traitement du peptomère par 2 mL de TFA à 25 % (v/v) dans du CH₂Cl₂ pendant 10 minutes à température ambiante. Le mélange réactionnel contenant le produit final a été dilué avec du CH₂Cl₂ (5 mL) avant l'évaporation complète sous vide. Afin d'éliminer toute trace de TFA, le produit dissous dans le CH₂Cl₂ a été évaporé sous vide, en répétant cette opération 5 fois.

### Procédures générales pour l'acylation de l'extrémité N-terminale du peptomère lié à la résine

*Acétylation* : À la résine a été ajoutée 1,56 mL d'une solution 1,7 M d'anhydride acétique dans le DMF (20,0 équivalents), suivie de 0,8 mL d'une solution 2,0 M de DIEA dans le DMF (12,0 équivalents). Après avoir été agitée pendant 90 minutes à 25 °C, la résine a été filtrée et lavée avec du DMF (5 × 2 mL).

*Benzoylation* : À la résine a été ajoutée 0,8 mL d'une solution 1.0 M de chlorure de benzoyle dans du CH₂Cl₂ (6,0 équiv.), suivie de 0,8 mL d'une solution 2,0 M de DIEA dans du CH₂Cl₂ (12,0 équiv.,). Après avoir été agitée pendant 40 minutes à 25 °C, la résine a été égouttée et lavée avec du CH₂Cl₂ (5 × 2 mL).

*Acylation à partir d'acides carboxyliques* : Dans une réaction de couplage typique décrite ici à partir de l'acide phénylacétique, 2,0 mL d'une solution 0,4 M d'acide phénylacétique dans le DMF (6,0 équivalents) et 2,13 mL d'une solution 0,5 M de DIC (8,0 équivalents) dans le DMF ont été ajoutées successivement à la résine. Après avoir été agitée pendant 1 h à 25 °C, la résine a été égouttée et lavée avec du DMF (5 × 2 mL).

Parmi les composés (YC55, YC-60) qui ont été synthétisés, YC55 a démontré une puissance égale à NUCC5953 pour perturber l'interaction TRIP8b-HCN (Tableau 1 : IC50 des peptoïdes).

**Tableau 1**

| | YC55 | YC60 | Acox3 |
|---|---|---|---|
| IC50 | 8.862 | 12.71 | 0.6029 |

L'effet analgésique de composés peptidomimétiques innovants injectés soit par voie intrathécale soit par voie systémique a été démontré dans la douleur neuropathique aiguë induite par l'oxaliplatine chez la souris.

Ainsi, le composé principal YC55 a démontré un effet analgésique dépendant de la dose chez les souris OIPN lorsqu'il est administré par voie i.t. (Figure 9).

Le composé YC55 a également démontré un effet analgésique dépendant de la dose chez les souris OIPN lorsqu'il est administré par voie s.c. (Figure 10).

Aucune altération de l'activité locomotrice n'a été observée lors du test Rotarod lorsque les souris sont traitées par YC55 administré par voie sous-cutanée (Figure 11).

YC55 a également démontré un effet analgésique dépendant de la dose dans un modèle de rat de neuropathie induite par le paclitaxel lorsqu'il est administré par voie i.t. (Figure 12).

L'administration intrathécale de YC55 a montré un effet antihypéralgique dans un modèle de neuropathie induite par une lésion traumatique du nerf sciatique (Figure 13), ce qui suggère que la stratégie de perturbation de l'interaction TRIP8b pourrait avoir un effet analgésique dans plusieurs états de douleur neuropathique.

Les composés MP208, MP405 (5µg, i.t.) et MP341, MP354, MP376 (10mg/kg, s.c.) ont démontré un effet analgésique dans un modèle de neuropathie induite par l'oxaliplatine chez la souris (Figure 14 et Figure 15 respectivement).

De même, les composés LF108, LF306, LF188 (Figure 16), LF400, LF329, et LF295 (Figure 17), LF275, LF261 et LF126 (Figure 18), LF239, LF222 et LF176 (Figure 19), ont démontré un effet analgésique (5µg, i.t.) dans un modèle de neuropathie aigüe induite par l'oxaliplatine chez la souris.

### B- Peptidomimétiques trimères

Les composés peptomères trimériques sont également des mimes de la séquence de l'extrémité carboxy-terminale du canal HCN₂. Ils ont été conçus en s'appuyant sur l'étude approfondie de la structure aux rayons X d'un co-cristal de TRIP8b avec la région C-terminale de HCN2 (pdb : 4EQF)

Ces ligands ont une formule qui est représentée par la formule LF369 ci-dessous ou la formule générale (II) suivante : 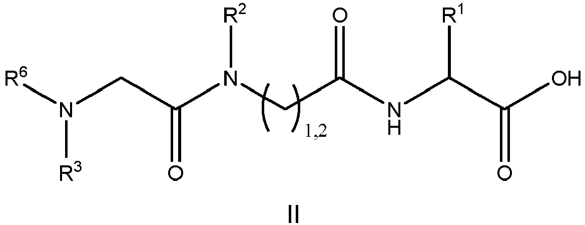 où
**R¹**, **R²** et **R³** sont tels que définis précédemment ;
**R⁶** est H, **R³**, CO**R⁵** (où **R⁵** est tel que défini précédemment), ou **R⁷** ;
**R⁷** est de manière indépendante un groupe parmi :

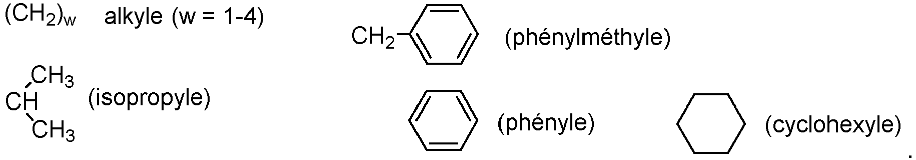

Les composés sont des oligomères hybrides peptide-peptoïde (peptomères) (Ostergaard et al., 1997) [17]. Ils comprennent trois unités dont un α-amino acide positionné à l'extrémité C-terminale de la séquence, suivi par deux résidus peptoïde.

Les synthèses ont été réalisées en solution à partir d'un acide α-aminé protégé sous forme d'ester de *tert*-butyle (Xaa-*t*Bu, la L-Leu-*t*Bu dans le cas de la synthèse de LF369). Ces synthèses peuvent également être réalisées sur résine comme précédemment décrit pour les peptidomimétiques tétramères. A partir de cette unité, l'élongation a été réalisée comme pour les molécules répondant à la formule générale (I), par des approches de "chimie sous-monomères ». Lorsque l'unité centrale est de type β-peptoïde, les deux étapes nécessaires à sa construction sont la réaction de Xaa-tBu avec le chlorure d'acryloyle, suivi d'une réaction aza-Michael permettant d'introduire la chaîne latérale souhaitée (**R²**) au squelette. La troisième unité α-peptoïde est construite par acylation du dimère avec le bromure de l'acide bromoacétique suivi de la substitution de l'atome de brome par une amine. Dans le cas où **R⁶** est H, l'amine utilisée est une amine primaire **R³**NH₂. Lorsque **R⁶** est CO**R⁵**, l'amine secondaire obtenue après traitement par **R³**NH₂ est acylée par un chlorure d'acide, un anhydride d'acide ou un acide carboxylique dans des conditions de couplage. Les composés pour lesquels **R⁶** = **R⁷** sont obtenus en traitant les dimères bromoacétylés à l'extrémité N-terminale par une amine secondaire **R³R⁷**NH. Ils peuvent également être obtenus par amination réductrice des composés dont l'extrémité N-terminale est NH**R³**. L'étape finale consiste à déprotéger les chaînes latérales et l'extrémité C-terminale par un traitement au TFA.

### Le protocole expérimental précis pour la synthèse de LF369 est le suivant :

Dans un ballon, la L-leu-tBu (1,74 g, 7,8 mmol) est dissoute dans 45 mL de THF anhydre. Le ballon sous atmosphère inerte d'argon est refroidi à 0°C. La triéthylamine (2,3 mL, 31,4 mmol, 4 équiv.) est ajoutée, suivi du chlorure d'acryloyle (0,76 mL, 9,4 mmol, 1,2 équiv). Après 3 h d'agitation à 0 °C une chromatographie sur couche mince (50/50 cyclohexane/AcOEt) indique une conversion totale. Les sels formés sont éliminés par filtration, le filtrat est évaporé sous vide et le produit obtenu est purifié par chromatographie sur gel de silice (50/50 cyclohexane/AcOEt). L'acrylamide est isolé avec un rendement de 93% (1,76 g, 7,27 mmol) sous la forme d'un solide blanc.

*Réaction aza-Michael.* L'acrylamide formé précédemment (1,76 g, 7,27 mmol) est dissout dans 20 mL d'éthanol. L'éthanolamine dont la fonction alcool a été protégée sous forme d'éther de tertbutyldiméthylsilyle (2,68 g, 15,28 mmol, 2,1 équiv.) est ajoutée et le mélange réactionnel est chauffé à reflux pendant 60 h. Le solvant est alors évaporé et le brut réactionnel purifié par chromatographie sur gel de silice (90/10/0,1 AcOEt/MeOH/Et₃N) ce qui permet l'obtention du dimère avec un rendement de 90 % (2,74 g, 6,58 mmol) sous forme d'une huile jaunâtre.

*Réaction d'acylation du dimère.* Le dimère (2,74 g, 6,58 mmol) est dissout dans 10 mL de THF, le réacteur est inerte à l'argon puis refroidi à -20°C. De la triéthylamine (1,1 mL, 7,89 mmol, 1,2 équiv.) est ajoutée à la solution, suivi de l'ajout de bromure de 2-bromoacétyle (0,62 mL, 7,89 mmol, 1,2 équiv.). Après 3 h d'agitation à -20 °C, le mélange réactionnel est filtré. Le filtrat obtenu est évaporé et le brut réactionnel est chromatographie sur gel de silice (60/40/0,1 Cyclohexane/AcOEt/Et₃N). Le dimère bromoacétylé est isolé pur avec un rendement de 67% (2,36 g, 4,4 mmol) sous forme d'une huile jaunâtre.

*Réaction de substitution.* Le produit précédemment obtenu (2,36 g, 4,4 mmol) est dissout dans 10 mL de THF. De la triéthylamine (1,2 mL, 8,8 mmol, 2 équiv.) puis l'éthanolamine protégée H₂NCH₂CH₂OTBDMS (2,31 g, 13,2 mmol, 3 équiv.) sont ajoutées à la solution. Le milieu réactionnel sous atmosphère d'argon est agité pendant une nuit à température ambiante. Le mélange réactionnel est alors filtré et le filtrat obtenu est évaporé. Le produit brut est purifié par chromatographie sur gel de silice (95/5/0.1 AcOEt/MeOH/Et₃N) conduisant au trimère avec un rendement de 83 % (2,30 g, 3,64 mmol) sous forme d'huile.

*Acétylation du trimère.* L'amine obtenue (2,3 g, 3,64 mmol) est dissoute dans 20 mL d'acétate d'éthyle. De la triéthylamine (2,02 mL, 14,5 mmol, 4 équiv.) puis de l'anhydride acétique (2,75 mL, 29 mmol, 8 équiv.) sont ajoutés à la solution. La réaction est placée sous argon, à température ambiante pendant 60 h. Après évaporation du solvant le brut obtenu est chromatographie sur gel de silice (90/10/0.1 AcOEt/cyclohexane/Et₃N) conduisant au trimère N-acétylé avec un rendement de 92 % (2,27 g, 3,36 mmol) sous forme d'huile incolore.

*Déprotection des chaînes latérales et de l'extrémité C-terminale.* Le composé trimérique (502 mg, 0,776 mmol, 1 équiv.) est dissout dans 4 mL d'un mélange TFA/ CH₂Cl₂/H₂O (47,5 : 47,5 : 5). Après 2 h d'agitation à température ambiante, 10 mL de dichlorométhane sont ajoutés. Le milieu réactionnel est évaporé sous vide puis 5 coévaporations au dichlorométhane sont effectuées. Le produit est ensuite redissout dans 5 mL d'eau distillée puis laissé sous agitation à température ambiante pendant 2 h. Le solvant est évaporé puis cinq coévaporations au toluène sont effectuées conduisant à LF369 (362 mg) sous forme d'huile.

### Chaînes latérales

**R¹**, **R²** et **R³** sont tels que définis précédemment ;
**R⁶** est **R³**, H, CO**R⁵** (où **R⁵** est tel que défini précédemment), ou **R⁷** ;
**R⁷** est de manière indépendante un groupe parmi :

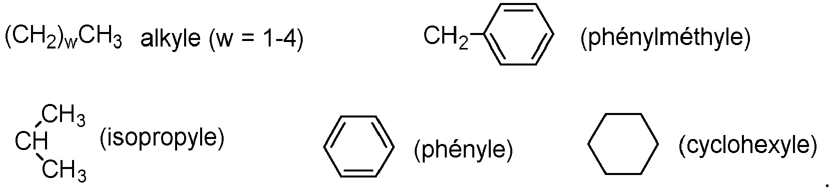

### Schéma synthétique représentatif de la synthèse en solution de peptoïdes de formule générale (II), e.g. LF369

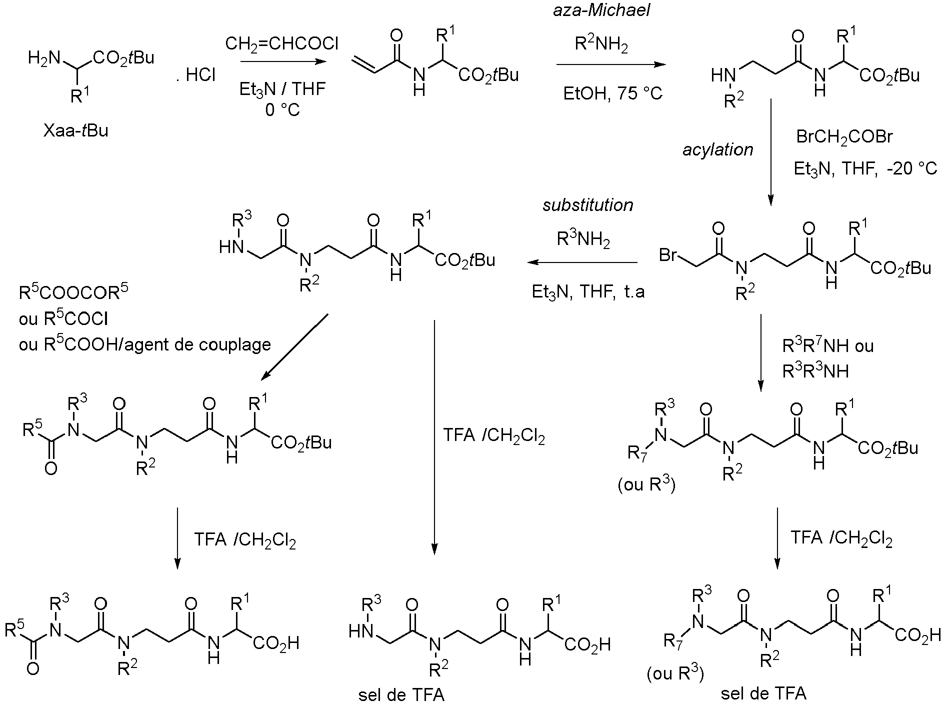

### Schéma synthétique représentatif de la synthèse supportée de peptoïdes de formule générale (II), e.g. LF369

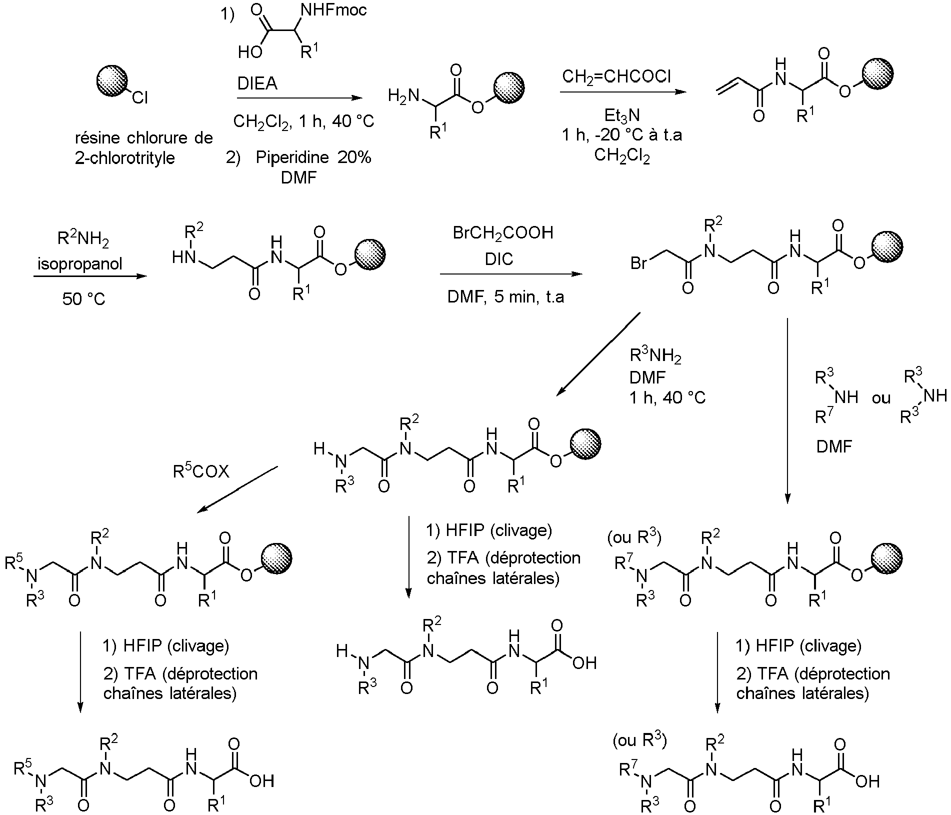

### Molécule synthétisée

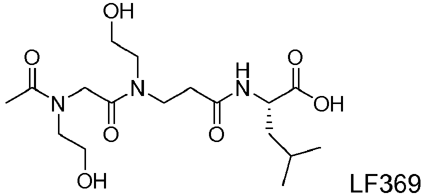

### Synthèse

Le composé LF369 (5 µg, i.t.) a démontré un effet analgésique dans un modèle de neuropathie induite par l'oxaliplatine chez la souris (Figure 20).

### Liste de Références

1. Finnerup et al., Lancet Neurol., 14(2) : 162-173, 2015
2. von Hehn et al., Neuron, 73(4) : 638-652, 2012
3. Hershman et al., J. Clin. Oncol., 32(18) : 1941-1967, 2014
4. Ludwig et al., Nature, 393(6685) : 587-591, 1998
5. Santoro et al., Cell, 93(5) : 717-729, 1998
6. Seifert et al., Proc. Natl. Acad. Sci. USA, 96(16) : 9391-9396, 1999
7. Dunlop et al., Curr. Pharm. Des., 15(15) : 1767-1772, 2009
8. Lewis et al., Mol. Cell Neurosci., 46(2) : 357-367, 2011
9. Descoeur et al., EMBO Mol. Med., 3(5) : 266-278, 2011
10. Young et al., Pain, 155(9) : 1708-1719, 2014
11. Lewis et al., J. Neurosci., 29(19) : 6250-6265, 2009
12. Bankston et al., Proc. Natl. Acad. Sci. USA, 109 : 7899, 2012
13. Han et al., J. Biomol. Screen, 20(9) : 1-8, 2015
14. Janssen et al., Arzneimittelforschung, 13 : 502-507, 1963
15. Randall et Selitto, Arch. Int. Pharmacodyn. Ther., 111(4) : 409-419, 1957
16. Kim et Chung, Pain, 50(3) : 355-363, 1992
17. Ostergaard et al., Molecular Diversity, 3 : 17-27, 1997(
18. Simon et al., Proc. Natl. Acad. Sci USA, 89 : 9367-9371, 1992
19. Hamper et al., J. Org. Chem., 63 : 708-718, 1998
20. Zuckermann et al., J. Am. Chem. Soc., 114 : 10646-10647, 1992
21. Culf et al., Molécules, 15 : 5282-5335, 2010
22. Miller et al., Drug Dev. Res., 35 : 20-32, 1995
23. Kwon et al., J. Am. Chem. Soc., 129 : 1508-1509, 2007
24. Vollrath et al., Organic & Biomolecular Chemistry, 11 : 8497-8201, 2013
25. Li et al., Cellular & Molecular Immunol., 7 : 133-142, 2010
26. Chongsiriwatana et al., Proc. Natl. Acad Sci. USA, 105 : 2794-2799, 2008
27. Mesirca et al., Nat. Commun., 5 : 4664.doi :10.1038/ncomms5664, 2014
28. Marger et al., Channels (Austin), 5(3) : 241-250, 2011
29. Hruby, Peptides, 116 : 63-67, 2019
30. Demande de Brevet EP 2289886

## Revendications

1. Peptoïde de formule générale (I) suivante : 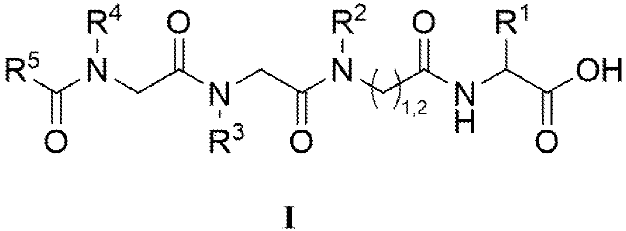 où
**R¹** est de manière indépendante CH₃ (L-Ala), CH(CH₃)₂ (L-Val), CH(CH₃)CH₂CH₃ (L-Ile), CH₂CH(CH₃)₂ (L-Leu and D-Leu), CH₂C(CH₃)₃, C(CH₃)₃, CH₂(cyclobutyl) ;
**R²** et **R³** sont choisis de manière indépendante parmi: CH₂CH(CH₃)₂, CH₂CH₂NH₂, CH₂CH₂OH, CH₂CH₂CH₂OH, CH₂CH(OH)CH₃, CH₂CONH₂, CH₂CH₂CONH₂ ;
**R⁴** est un groupe aliphatique ou aromatique choisi parmi : 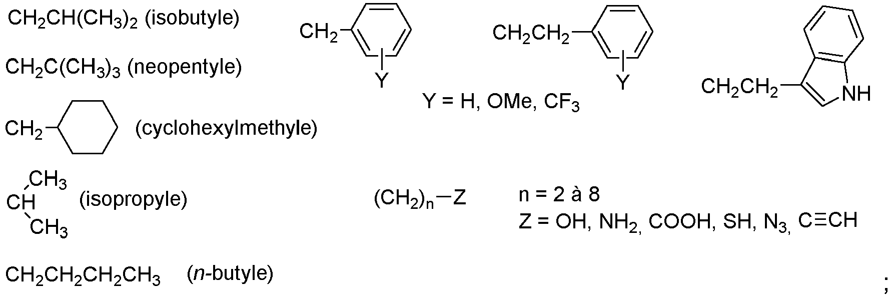
**R⁵** est de manière indépendante CH₃, CH(CH₃)₂, CH₂C₆H₅, C₆H₄X (où X en position ortho, méta ou para est de manière indépendante H, OMe, CF₃, CH₃, CH₂CH₃, F, Br, Cl, NO₂), 3-indolyl, 3-quinolinyl.

2. Peptoïde selon la revendication 1 de formules suivantes :

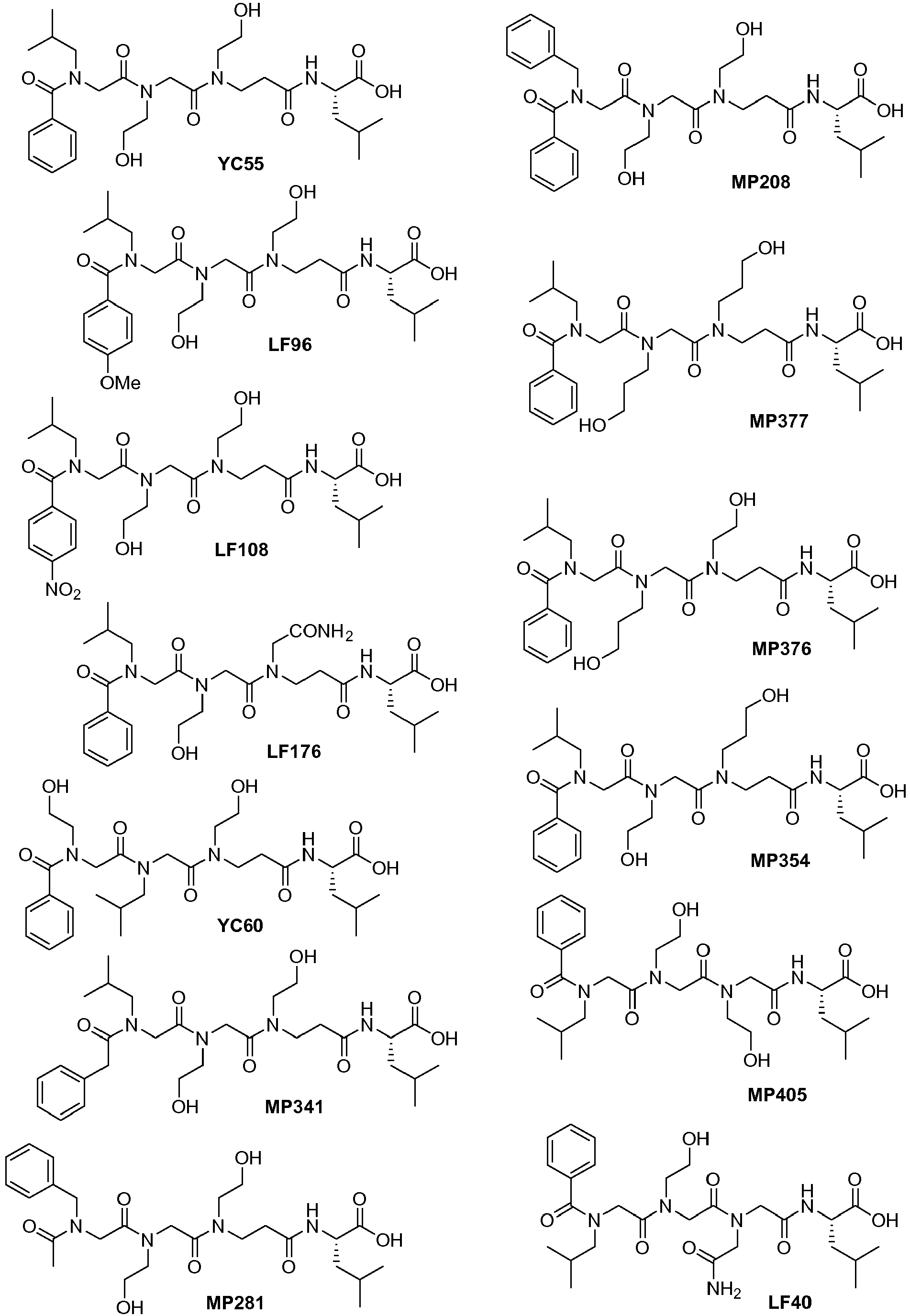

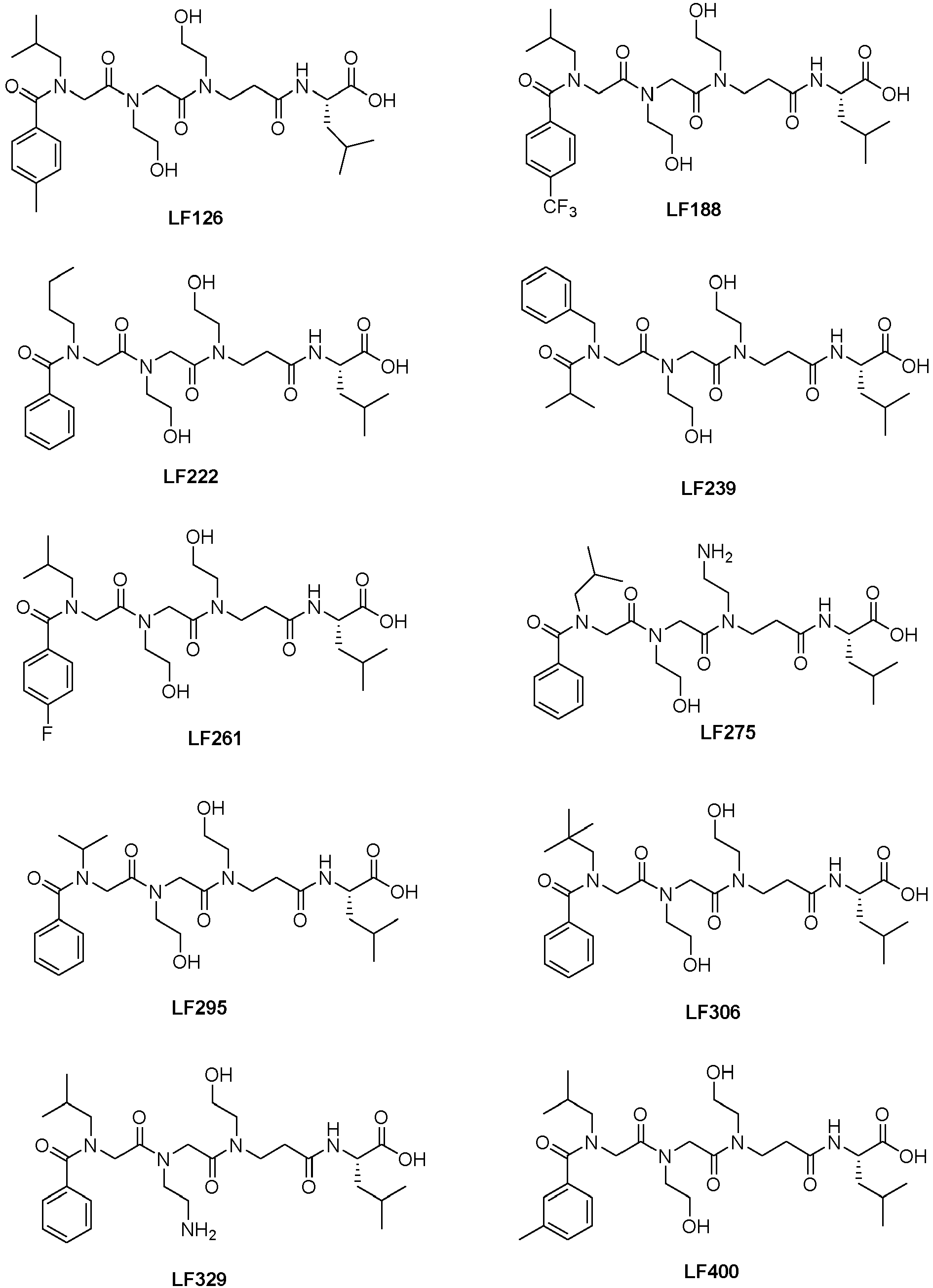

3. Peptoïde de formule générale (II) suivante : 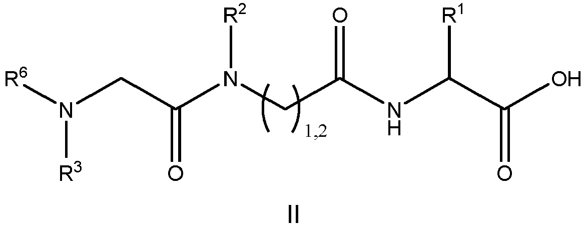 où
**R¹**, **R²** et **R³** sont tels que définis dans la revendication 1 ;
**R⁶** est **R³**, H, CO**R⁵** (où **R⁵** est tel que défini ci-dessus) ou **R⁷**;
**R⁷** est de manière indépendante un groupe parmi :

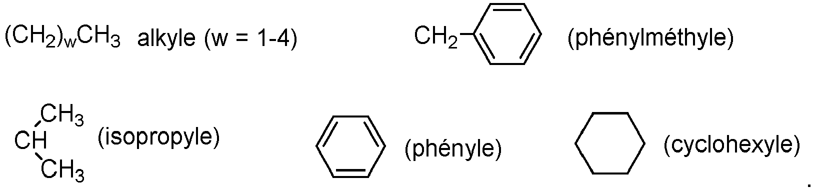

4. Composition pharmaceutique comprenant un peptoïde selon l'une quelconque des revendications 1 à 3, et un véhicule pharmaceutiquement acceptable.

5. Peptoïde selon l'une quelconque des revendications 1 à 3 ou composition pharmaceutique selon la revendication 4, pour une utilisation comme médicament.

6. Peptoïde selon l'une quelconque des revendications1 à 3 ou composition pharmaceutique selon la revendication 4, pour une utilisation dans la prévention ou le traitement de la douleur chronique.

7. Peptoïde pour une utilisation selon la revendication 6, où la douleur chronique est d'origine neuropathique.

## Patentansprüche

1. Peptoid der folgenden allgemeinen Formel (I): 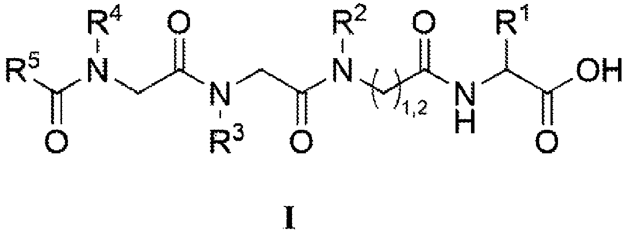 wobei
**R¹** unabhängig CH₃ (L-Ala), CH(CH₃)₂ (L-Val), CH(CH₃)CH₂CH₃ (L-Ile), CH₂CH(CH₃)₂ (L-Leu und D-Leu), CH₂C(CH₃)₃, C(CH₃)₃, CH₂(Cyclobutyl) ist;
**R²** und **R³** unabhängig ausgewählt sind aus: CH₂CH(CH₃)₂, CH₂CH₂NH₂, CH₂CH₂OH, CH₂CH₂CH₂OH, CH₂CH(OH)CH₃, CH₂CONH₂, CH₂CH₂CONH₂;
**R⁴** eine aliphatische oder aromatische Gruppe ist, ausgewählt aus: 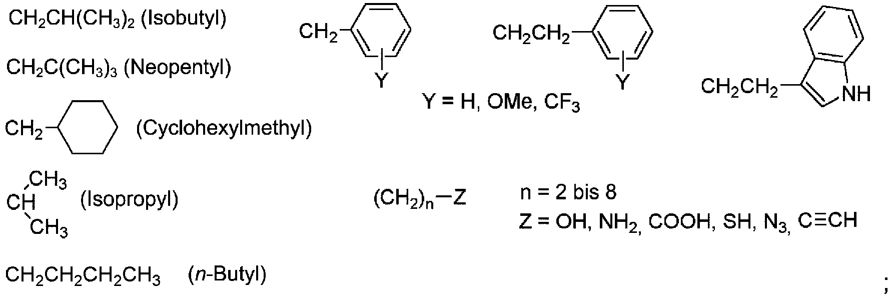
**R⁵** unabhängig CH₃, CH(CH₃)₂, CH₂C₆H₅, C₆H₄X (wobei X in der Ortho-, Meta- oder Para-Position unabhängig H, OMe, CF₃, CH₃, CH₂CH₃, F, Br, Cl, NO₂ ist), 3-Indolyl, 3-Chinolinyl ist.

2. Peptoid nach Anspruch 1 der folgenden Formeln:

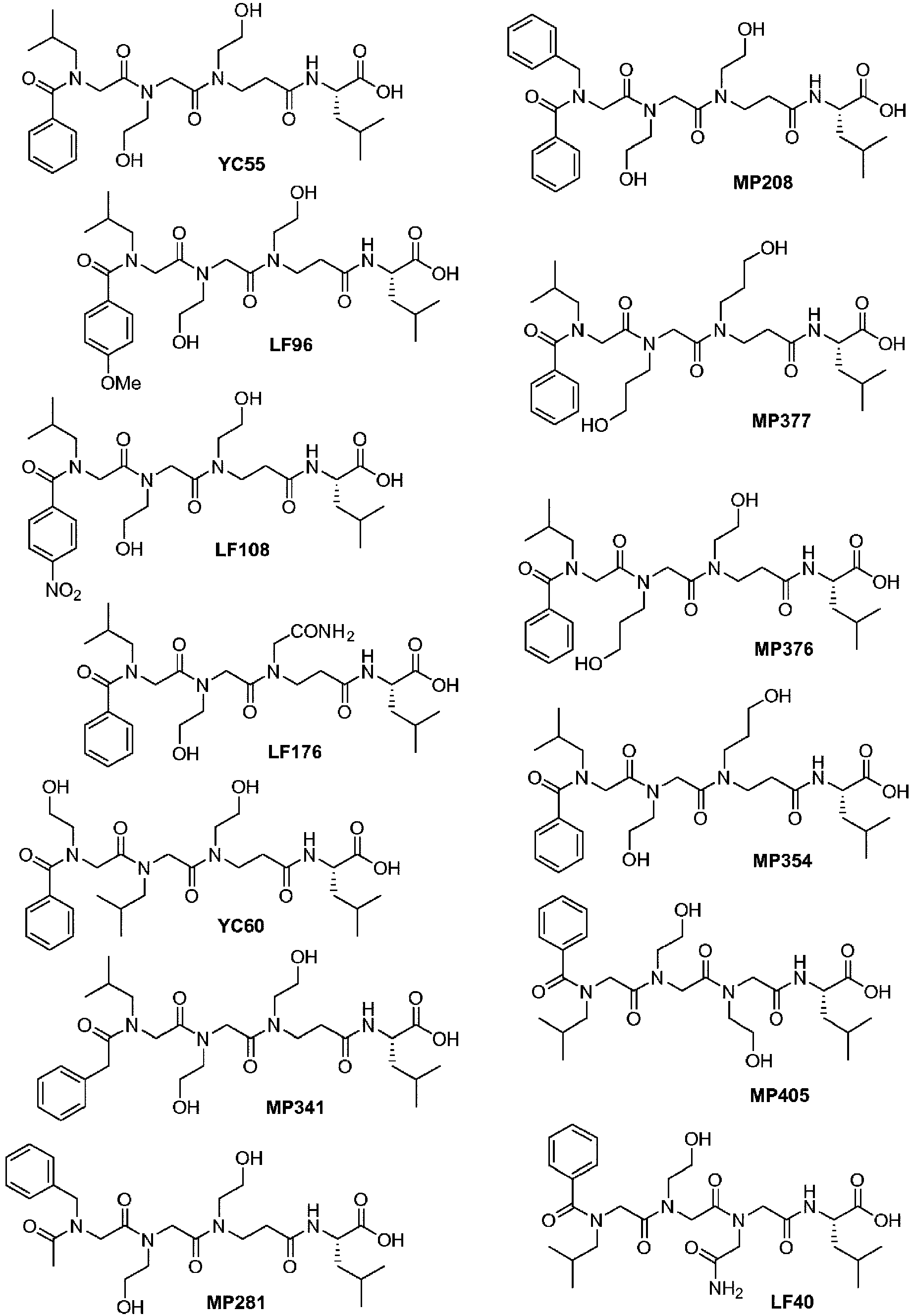

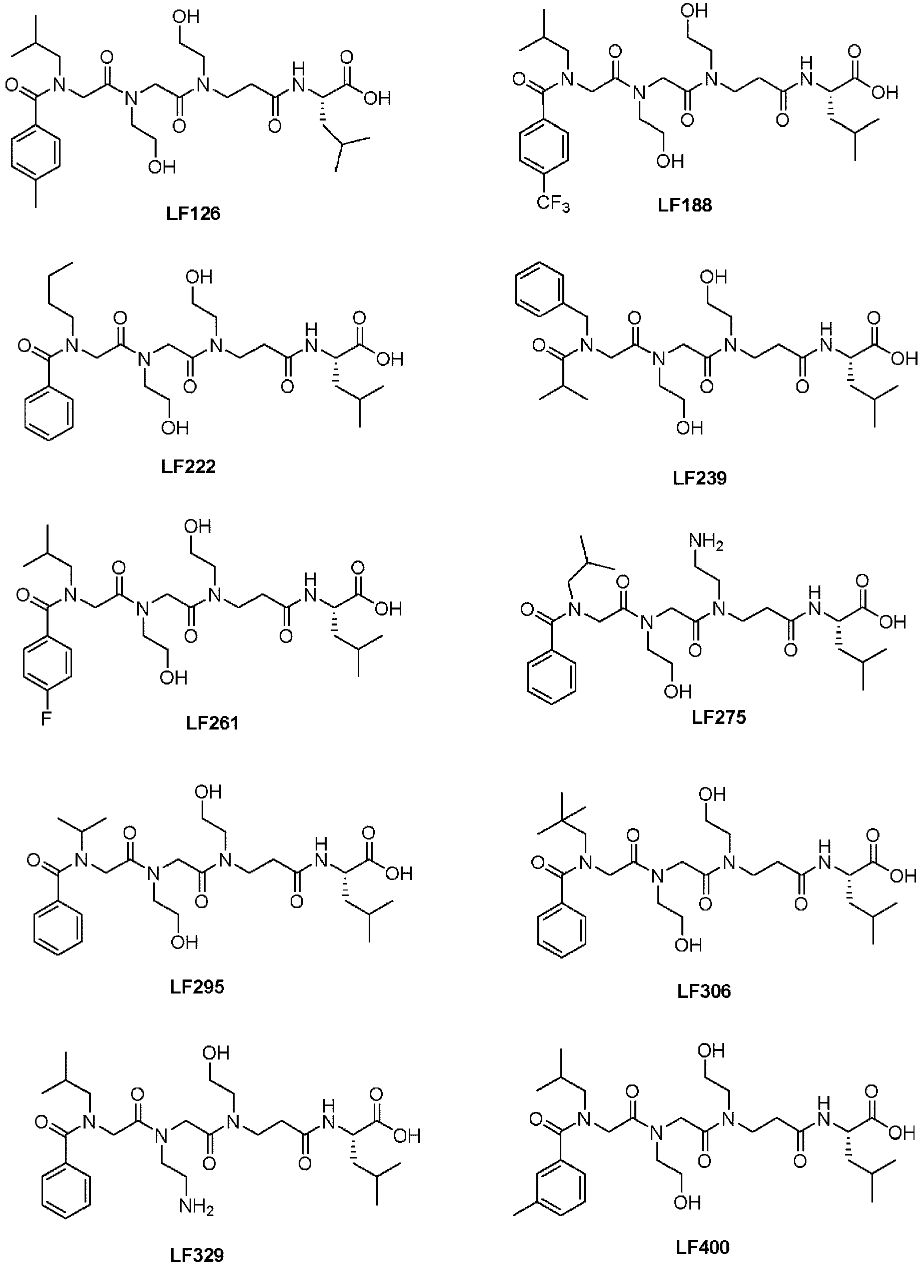

3. Peptoid der folgenden allgemeinen Formel (II): 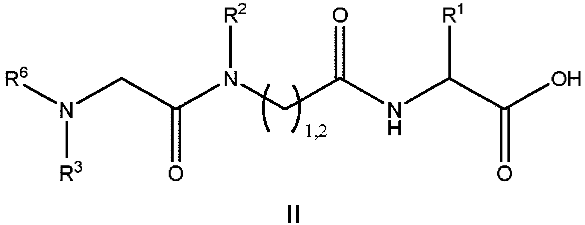 wobei
**R¹**, **R²** und **R³** wie in Anspruch 1 definiert sind;
**R⁶ R³,** H, CO**R⁵** (wobei **R⁵** wie oben definiert ist) oder **R⁷** ist;
**R⁷** unabhängig eine Gruppe ist aus:

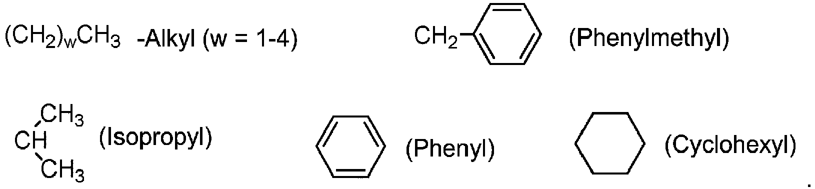

4. Pharmazeutische Zusammensetzung, umfassend ein Peptoid nach einem der Ansprüche 1 bis 3 und einen pharmazeutisch verträglichen Träger.

5. Peptoid nach einem der Ansprüche 1 bis 3 oder pharmazeutische Zusammensetzung nach Anspruch 4 für eine Verwendung als ein Arzneimittel.

6. Peptoid nach einem der Ansprüche 1 bis 3 oder pharmazeutische Zusammensetzung nach Anspruch 4 für eine Verwendung bei der Vorbeugung oder der Behandlung chronischer Schmerzen.

7. Peptoid für eine Verwendung nach Anspruch 6, wobei die chronischen Schmerzen neuropathischen Ursprungs sind.

## Claims

1. Peptoid of the following general formula (I): 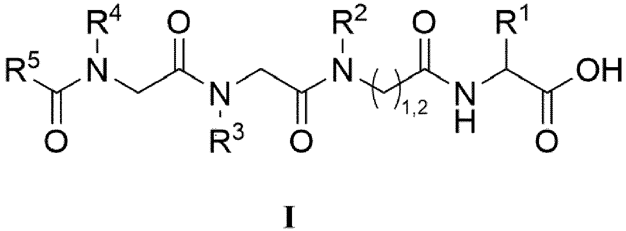 where
**R¹** is independently CH₃ (L-Ala), CH(CH₃)₂ (L-Val), CH(CH₃)CH₂CH₃ (L-Ile), CH₂CH(CH₃)₂ (L-Leu and D-Leu), CH₂C(CH₃)₃, C(CH₃)₃, CH₂(cyclobutyl);
**R²** and **R³** are independently selected from: CH₂CH(CH₃)₂, CH₂CH₂NH₂, CH₂CH₂OH, CH₂CH₂CH₂OH, CH₂CH(OH)CH₃, CH₂CONH₂, CH₂CH₂CONH₂ ;
**R⁴** is an aliphatic or aromatic group selected from: 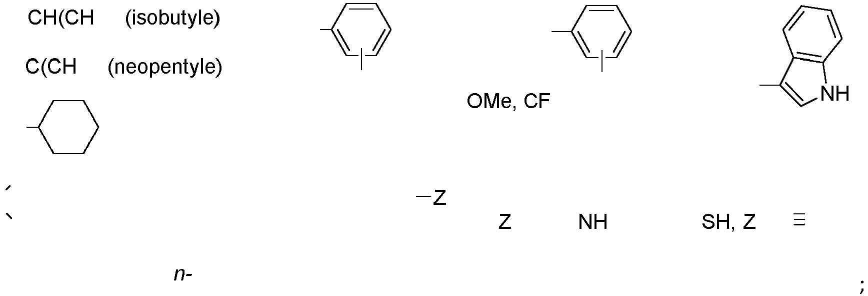
**R⁵** is independently CH₃, CH(CH₃)₂, CH₂C₆H₅, C₆H₄X (where X in the ortho, meta or para position is independently H, OMe, CF₃, CH₃, CH₂CH₃, F, Br, Cl, NO₂), 3-indolyl, 3-quinolinyl.

2. Peptoid according to claim 1 of the following formula:

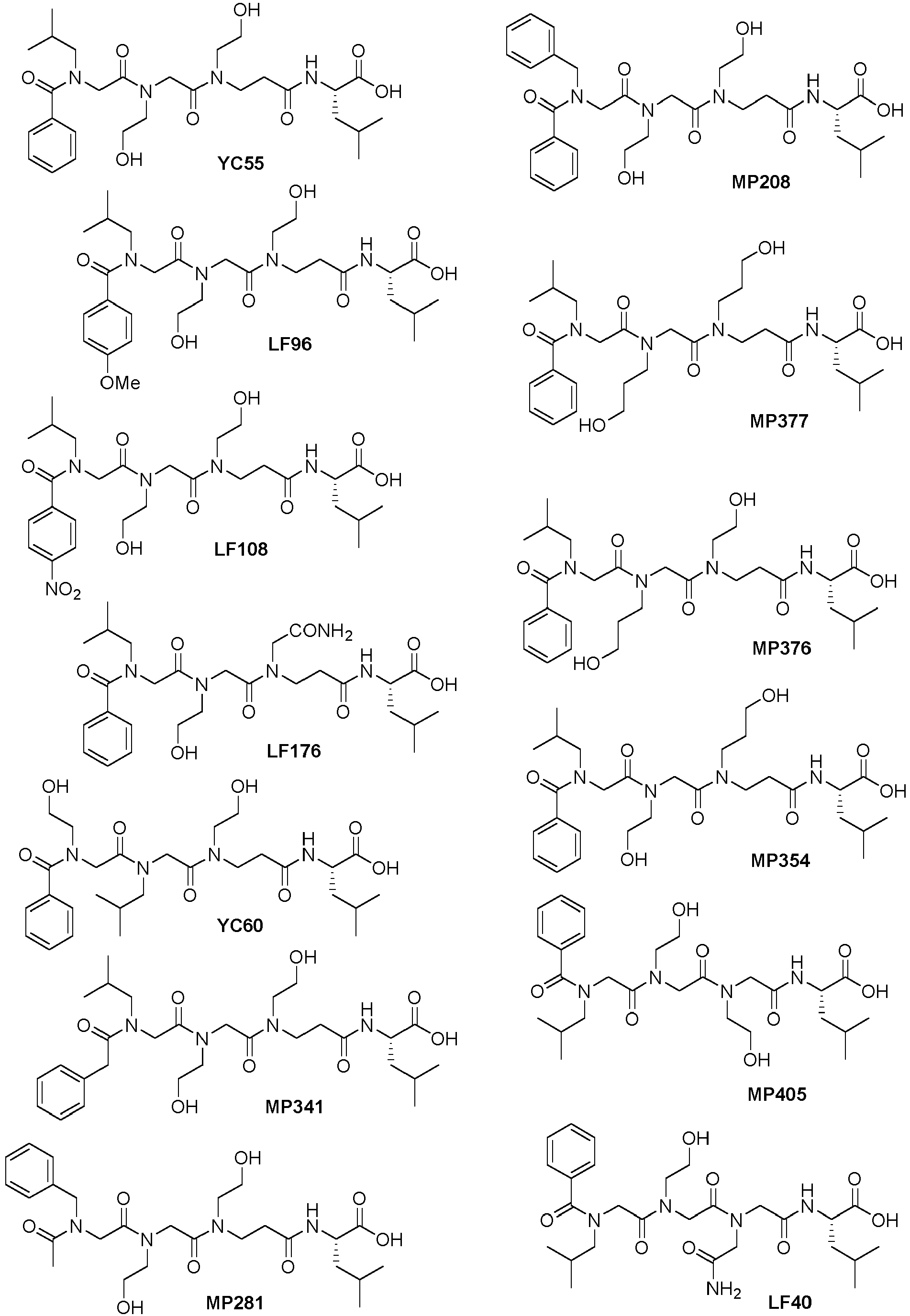

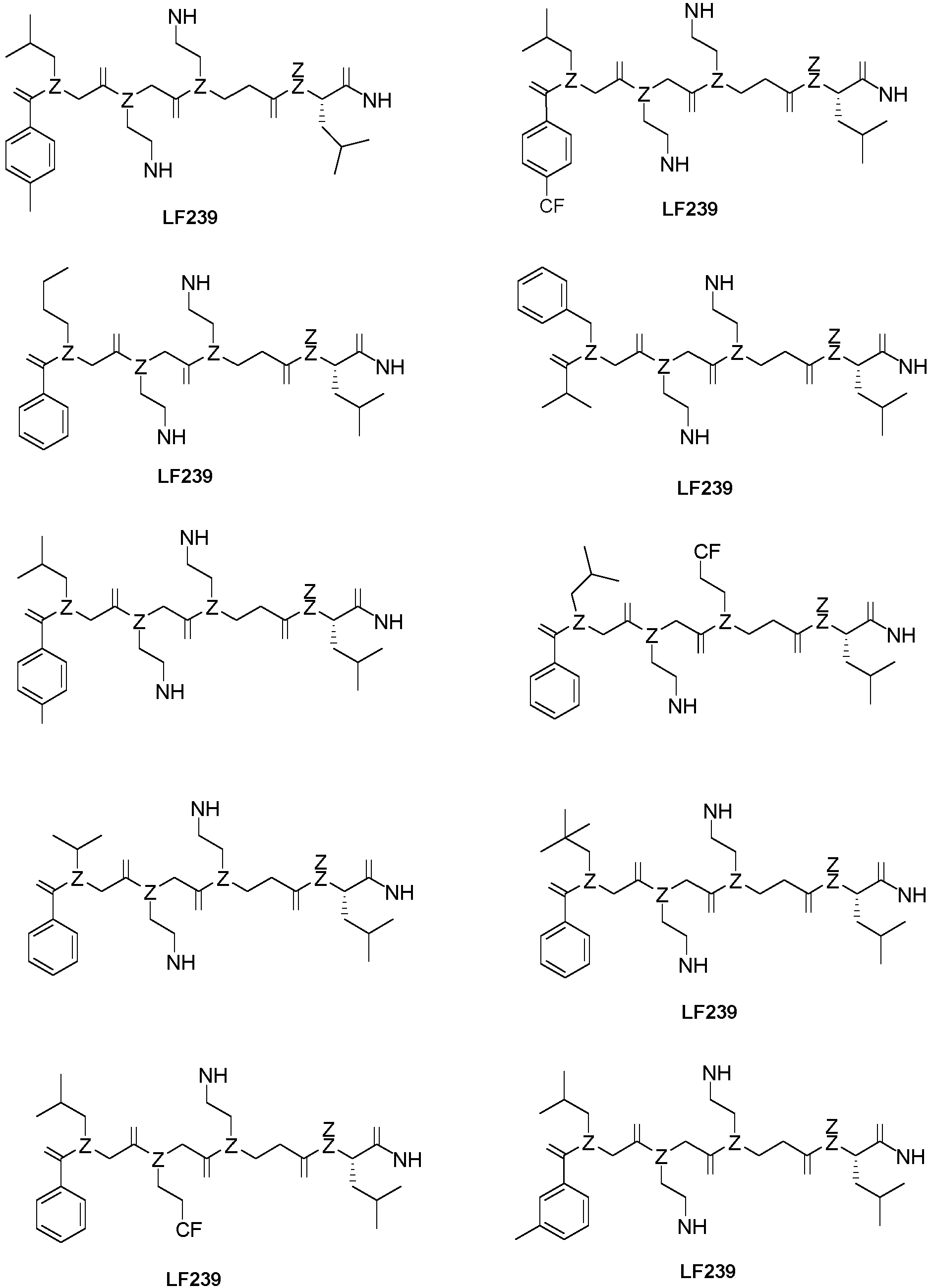

3. Peptoid of the following general formula (II): 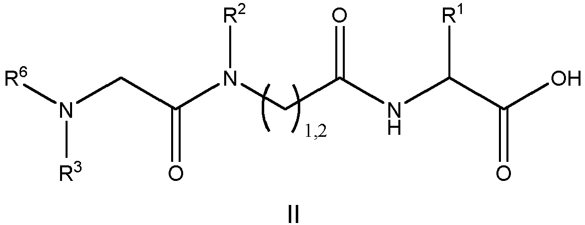 wherein
**R¹**, **R**² and **R³** are as defined in claim 1;
**R⁶** is **R³**, H, CO**R⁵** (wherein **R⁵** is as defined above) or **R⁷** ;
**R⁷** is independently one of:

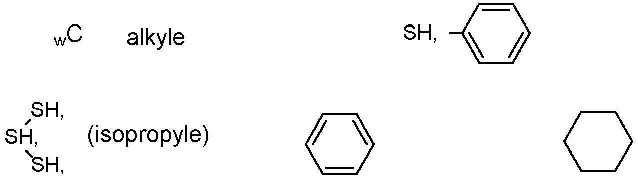

4. A pharmaceutical composition comprising a peptoid of any one of claims 1 to 3, and a pharmaceutically acceptable carrier.

5. The peptoid of any one of claims 1 to 3 or the pharmaceutical composition of claim 4, for use as a medicament.

6. The peptoid of any one of claims 1 to 3 or the pharmaceutical composition of claim 4, for use in the prevention or treatment of chronic pain.

7. A peptoid for use according to claim 6, wherein the chronic pain is of neuropathic origin.
